(19) Europäisches Patentamt
European Patent Office
Office européen des brevets

(11) **EP 1 663 274 B1**

(12) **EUROPÄISCHE PATENTSCHRIFT**

(45) Veröffentlichungstag und Bekanntmachung des Hinweises auf die Patenterteilung:
**28.03.2007 Patentblatt 2007/13**

(21) Anmeldenummer: **03818689.6**

(22) Anmeldetag: **20.08.2003**

(51) Int Cl.:
**A61K 36/899** (2006.01)   **A23L 1/10** (2006.01)

(86) Internationale Anmeldenummer:
**PCT/EP2003/009239**

(87) Internationale Veröffentlichungsnummer:
**WO 2005/027946 (31.03.2005 Gazette 2005/13)**

(54) **PFLANZLICHE PLAZENTA-EXTRAKTE, VERFAHREN ZU IHRER HERSTELLUNG UND IHRE VERWENDUNG**

VEGETAL PLACENTA EXTRACTS, METHOD FOR THE PRODUCTION AND USE THEREOF

EXTRAITS PLACENTAIRES VEGETAUX, PROCEDE POUR LES PREPARER ET LEUR UTILISATION

(84) Benannte Vertragsstaaten:
**CH DE LI**

(43) Veröffentlichungstag der Anmeldung:
**07.06.2006 Patentblatt 2006/23**

(73) Patentinhaber: **Riemschneider, Randolph, Dr. Dr. Prof.**
**14052 Berlin (DE)**

(72) Erfinder:
• **RIEMSCHNEIDER, Randolph, Prof. Dr. Dr.**
**14052 Berlin (DE)**
• **REIHER, Christel**
**10783 Berlin (DE)**

(74) Vertreter: **Hartmann, Günter**
**RUSCHKE HARTMANN MADGWICK & SEIDE**
**Patent- und Rechtsanwälte**
**Postfach 86 06 29**
**81633 München (DE)**

(56) Entgegenhaltungen:
EP-A- 0 552 516      WO-A-02/15711
CH-A- 692 408      FR-A- 2 786 778

• LING WEN HUA ET AL: "Supplementation of the black rice outer layer fraction to rabbits decreases atherosclerotic plaque formation and increases antioxidant status" JOURNAL OF NUTRITION, WISTAR INSTITUTE OF ANATOMY AND BIOLOGY, PHILADELPHIA, PA,, US, Bd. 132, Nr. 1, Januar 2002 (2002-01), Seiten 20-26, XP002245979 ISSN: 0022-3166
• DATABASE WPI Section Ch, Week 199522 Derwent Publications Ltd., London, GB; Class B05, AN 1995-166347 XP002268009 & JP 07 087930 A (OTSUKA SHOKUHIN KK), 4. April 1995 (1995-04-04)
• DATABASE WPI Section Ch, Week 200229 Derwent Publications Ltd., London, GB; Class B04, AN 2002-233881 XP002268010 & KR 2001 096 669 A (COSMECCA KOREA CO LTD), 8. November 2001 (2001-11-08)
• PATENT ABSTRACTS OF JAPAN vol. 1999, no. 01, 29. Januar 1999 (1999-01-29) & JP 10 287525 A (PURE:KK), 27. Oktober 1998 (1998-10-27)
• BAUBLIS A ET AL: "Antioxidant effect of aqueous extracts from wheat based ready-to-eat breakfast cereals" FOOD CHEMISTRY, Bd. 68, Nr. 1, Januar 2000 (2000-01), Seiten 1-6, XP002268008 ISSN: 0308-8146

**Beschreibung**

Die Erfindung betrifft ein Verfahren zur Herstellung von Cereallen-Plazenta-Extrakten aus Plazenta-Zelllinien der jeweiligen Cerealien, die dabei erhaltenen Produkte sowie deren Verwendung einzeln oder in Form von Kombinationen in pharmazeutischen, kosmetischen, diätetischen und sonstigen Zusammensetzungen, insbesondere in Form von sie (unverdünnt oder verdünnt) enthaltenden Kapseln für die orale Einnahme Insbesondere als Nahrungsergänzungsmittel.

Tierische Organextrakte, insbesondere tierische Plazenta-Extrakte, haben als Grundstoffe für Pharmaka und Kosmetika eine große Bedeutung. Insbesondere tierische Plazenta-Extrakte werden aufgrund der sie enthaltenden Wirkstoffe in Pharmaka und Kosmetika in großem Umfang eingesetzt [vgl. "Selfen-Öle-Fette-Wachse" 112, 211-218 (1986)], wobei sie in vielen Fällen gegebenenfalls noch durch niedermolekulare Zusätze ergänzt werden. In manchen Ländern, beispielsweise in Japan, ist die Anwendung tierischer Organextrakte nur noch bedingt erlaubt, beispielsweise Extrakte auf Basis von Schweineplazenten; letztere können in einigen Fällen die ab 12.12.2000 gesetzlich verbotenen Rinderplazenten-Extrakte ersetzen [R. Riemschneider, M. Heisler, Relata Techica Web Site htpp://www.vevy.com/relata, Isuues, Articles 2001, 3 Seiten].

Tierische Organextrakte, insbesondere tierische Plazenta-Extrakte, zeichnen sich durch eine Vielzahl von Inhalts- bzw. Wirkstoffen aus, die entweder in Form des vollständigen Extrakts oder in Form von daraus Isollerten Einzelsubstanzen Verwendung finden. Für tierische Pflanzen-Extrakte wurden beispielsweise über 100 Komponenten nachgewiesen [vgl. "Kosmetik International" 6, 1-8 (1978)]. Für einen Teil bestimmter Einzelsubstanzen der tierischen Plazenta-Extrakte ist die Synthese bisher nicht gelungen, zumal der dazu nötige Aufwand zu groß wäre.

Zahlrelche tierische Organextrakte, insbesondere Plazenta-Extrakte, stehen heute in mehr oder minder naturbelassener Form zur Verfügung, beispielsweise die oben erwähnten Schweineplazenta-Extrakte. Zu ihrer Herstellung wird das frische Organmaterial gewaschen, zerkleinert (zuletzt in Kolloidmühlen) und durch Behandeln des dabei erhaltenen Organmaterialbreis bei -10 bis -20 °C mit organischen Lösungsmitteln extrahiert.

Nach der Gewinnung der oben erwähnten wässrigen tierischen Rohextrakte durch Zentrifugieren bzw. Filtrieren oder andere geeignete Maßnahmen werden diese weiterverarbeitet zu reinen proteinhaltigen oder proteinfreien Extrakten, die bis zur weiteren Verwendung sterlifiltriert werden, beispielsweise unter Verwendung eines 0,2 $\mu$m Millipore®-Filters. Solche Präparate enthalten zuweilen noch Proteine oder hochmolekulare Protein-Abbauprodukte, die pathogene und immunogene Eigenschaften aufweisen können, und die bei regeimäßiger oder wiederholter Verabreichung - meistens verbunden mit langen inkubationszeiten - zu schwerwiegenden Erkrankungen führen können. Im Falle tierischer Organextrakte, insbesondere tierischer Plazenta-Extrakte, sind insbesondere die durch unkonventionelle Viren, d.h. Prionen, verursachten Krankheitsbilder bekannt, wie z.B. TSE (transmissible spongiforme Encephalopathien), insbesondere BSE (Rinderwahnsinn), und das Jacob-Creutzfeld-Syndrom. Es sind aber auch Protein-haltige tierische Plazenta-Extrakte bekannt, die bei ihrer Anwendung auf den Menschen krebserregend sein können.

Vor allem durch die mit Prionen, insbesondere mit BSE-Prionen, zusammenhängenden Probleme sind tierische Organextrakte, insbesondere tierische Plazenta-Extrakte, weitweit in Verruf geraten. Der Nachweis, dass Organmaterial von Herden stammt, die BSE-frei waren, ist im Falle der Verwendung großer Organmengen bestimmten Alters kaum durchzuführen. Um BSE-Freiheit eines Endprodukts experimentell im Goldhamster-Modell-Versuch zu beweisen, bedarf es mindestens 9 Monate Zeit - in vielen Fällen undurchführbar. Ein sicherer BSE-Schnelltest fehlt bisher.

Aus DE-OS 24 05 983 und DE-OS 20 21 969 sind Kombinationen niedermolekularer Komponenten mit aus natürlichem, frischem Organmaterial gewonnenen tierischen Organextrakten bekannt, die jedoch ebenfalls die vorstehend geschilderten Nachteile haben.

Um solche Komplikationen zu vermeiden, wurde in EP-A-0 552 516 ein Weg zur Herstellung wasserlöslicher synthetischer tierischer Organextrakte aufgezeigt, bei dem auf synthetischem Wege gewonnene niedermolekulare Komponenten, wie sie auch in natürlichen tierischen Organextrakten vorkommen, miteinander kombiniert werden.

Während die natürlichen tierischen Organextrakte die oben geschilderten Nachteile aufweisen, haben die synthetischen tierischen Organextrakte den Nachteil, dass sie großenteils den vollen biochemischen Wirkungsgrad der natürlichen tierischen Organextrakte, insbesondere der tierischen Plazenta-Extrakte, nicht entwickeln, weil ihnen bestimmte Komponenten fehlen.

Man ist daher seit langem bemüht, die in Verruf geratenen tierischen Organextrakte, insbesondere Plazenta-Extrakte, durch entsprechende pflanzliche Extrakte zu ersetzen, welche die oben genannten Nachteile nicht aufweisen.

In CH-A-692 408 ist ein Verfahren zur Gewinnung von Pflanzenextrakten und deren Verwendung als pflanzliches kosmetisches Additiv im Sinne von Kollagen-Ersatz (vgl. z.B. Tabelle VI) beschrieben. Dabei handelt es sich jedoch nicht um pflanzliche Plazenta-Extrakte.

In WO-A-02/15711 sind Verfahren zur Gewinnung von Aleuron aus Klele beschrieben. Dabei geht man von "gemischten" Zellverbänden mit inhomologer Zusammensetzung aus. Pflanzliche Plazenta-Zelllinien sind darin nicht beschrieben.

In dem Artikel von Ling Wen Hua et al., "Supplementation of the black rice outer layer fraction to rabbits decreases atherosclerotic plaque formation and increases antioxidant status", veröffentlicht im "Journal of Nutrition, Wistar Institute of Anatomie and Biology", Philadelphia, GA, US, Band 132, Nr. 1, Januar 2002, Seiten 20 bis 26, sind Extrakte von aus den äußeren Schichten von Relskörnern beschrieben. Dabei handelt es sich jedoch ebenfalls um gemischte Zellverbände mit inhomologer Zusammensetzung.

Dies gilt auch für die Veröffentlichungen in "DataBase WPI Section Ch, Week 199522 Derwent Publications", Ltd., London, GB, AN 1995-166 347 XP 00 226 809 + JP-A-07/087 930 sowie in "Patent Abstracts of Japan", Band 199, Nr. 01, 29. Januar 1999 + JP-A-10/287 525, die sich auf Präparate beziehen, die aus der Hülle von Cerealien hergestellt werden. Auch hierbei handelt es sich um gemischte Zellverbände mit inhomologer Zusammensetzung.

In einem Artikel von R. Rlemschneider, "Cytocatalyzer, Cellryel and Collaplant PO, plant-based cosmetic additives", veröffentlicht im Internet durch das "Bimonthly Journal of the BWW Society", am 29.06.03, ist ein pflanzlicher Plazenta-Extrakt aus Roggen (Secale cereale) beschrieben, der durch mehrere Extraktionen aus Zellkulturen gewonnen wird. Auch in diesem Fall geht man von gemischten Zellverbänden aus unterschiedlichen Teilen der Roggen-Plazenta mit inhomologer Zusammensetzung aus. Außerdem enthält dieser Artikel keine Einzelheiten hinsichtlich der Durchführung des Herstellungsverfahrens.

Der in der vorliegenden Anmeldung beschriebenen Erfindung liegt die Aufgabe zugrunde, Plazenta-Extrakte von Cerealien zur Verfügung zu stellen, die das biochemische Wirkungsprofil von natürlichen Plazenta-Extrakten und gemischten Zellkultur-Extrakten dieser Cerealien mindestens erreichen und vorzugsweise übertreffen, andererseits aber zuverlässig reproduzierbar frei von unerwünschten Substanzen, insbesondere immunogenen und pathogenen Proteinen und Protein-Abbauprodukten, und daher auch frei von deren unerwünschten Wirkungen sind wie synthetische Plazenta-Extrakte von Cerealien, ohne jedoch deren Nachteile in Bezug auf ihr ungünstiges biologisches/biochemisches Wirkungsprofil zu besitzen.

Es wurde überraschend gefunden, dass diese Aufgabe auf technisch einfache und außerordentlich wirkungsvolle Weise dadurch gelöst werden kann, dass als Ausgangsmaterial für die Herstellung von Cerealien-Plazenta-Extrakten mit verbessertem biochemischem Wirkungsgrad, die frei von unerwünschten Substanzen und in beliebier Menge produzierbar sind, Zelllinien, d.h. Klone (genetisch identische Individuen) von Einzelzellen der Plazenta spezifischer Cerealien verwendet werden, die im Gegensatz zu gemischten Zellkulturen der Cerealien-Plazenta, die aus Zellen unterschiedlichen Typs bestehen, in großtechnischem Maßstab beliebig oft und in beliebiger Menge unter Erzielung stets gleichbleibender, zuverlässig reproduzierbarende Eigenschaften vermehrt und damit zur Massenproduktion von gesundheitlich unbedenklichen und dennoch wirksamen Plazenta-Extrakten der gewünschten spezifischen Cerealien eingesetzt werden. Das wesentliche Prinzip, das der in der vorliegenden Anmeldung beschriebenen Erfindung zugrunde liegt, besteht darin, anstelle der ganzen Plazenten oder anstelle von gemischten Zellverbänden der Plazenten von Cerealien, Cerealien-Plazenta-Zelllinien mit Ihren zuverlässig reproduzierbaren Eigenschaften und Wirkungen einzusetzen. Dies führt zu überraschenden, biologisch-technischen Vorteilen, deren Bedeutung gar nicht hoch genug eingeschätzt werden kann, insbesondere wenn man berücksichtigt, dass viele Mechanismen der in den Zellen ablaufenden biologisch-chemischen Vorgänge noch gar nicht oder nicht ausreichend bekannt sind. Es ist daher von höchster Bedeutung, ein zuverlässig reproduzierbares Ausgangsmaterial mit zuverlässig reproduzierbaren Eigenschaften einzusetzen, dessen Wirkungen abschätzbar und stets gleichbleibend sind, ohne den genauen Mechanismus, der im einzelnen ablaufenden biologisch-chemischen Vorgänge zu kennen.

Zur Durchführung des hier beschriebenen Verfahrens können im Prinzip alle bereits bekannten Cerealien-Plazenta-Zelllinien eingesetzt werden, sowohl solche, die eigens dafür hergestellt worden sind, als auch solche, die bei einschlägig anerkannten Instituten hinterlegt sind, als auch noch nicht bekannte und noch nicht bei allgemein anerkannten Instituten hinterlegte Cerealien-Plazenta-Zelllinien und künftig noch zu entwickelnde neue Cerealien-Plazenta-Zelllinien. Die vom Anmelder mit bestimmten Cereallen-Plazenta-Zelllinien bereits festgestellten überraschenden Effekte und die für andere Cerealien-Plazenta-Zelllinien vom Fachmann zu erwartenden Effekte führen zu einem beträchtlichen technischen Fortschritt, der kein Vorbild im Stand der Technik hat und daher auch vom Fachmann auf diesem Gebiet nicht vorhersehbar war, wie die zahlenmäßigen Nachweise in den weiter unten folgenden Ausführungsbelsplelen zeigen.

Die vorliegende Erfindung betrifft somit ein Verfahren zur Herstellung von Plazenta-Extrakten von spezifischen Cerealien aus Plazenta-Zellen von Zelllinien dieser Cerealien, wie sie in den Patentansprüchen 1 bis 8 beschrieben sind, sowie deren Verwendungen auf verschiedenen Einsatzgebieten entsprechend den Patentansprüchen 9 bis 17, deren charakteristisches Merkmal darin besteht, dass sie aus Plazenta-Zellen von Zeillinien vom Cerealien aus der Gruppe Weizen, Reis, Mais, Gerste, Roggen, Hafer, Sorghum (Hirse), Triticale und Dinkel hergestellt sind, die aus frischem Cerealien-Plazenta-Materlal gewonnen worden sind oder von einer Zellbank stammen, in der sie hinterlegt und frei zugänglich sind, sodass sie von beliebigen Dritten von der genannten Zellbank bezogen werden können.

Gegenstand der vorliegenden Erfindung ist gemäß einem ersten Aspekt ein Verfahren zur Herstellung von Plazenta-Extrakten von Cerealien aus der Gruppe Weizen, Rels, Gerste, Roggen, Hafer, Sorghum (Hirse), Triticale (eine Kreuzung aus Weizen und Roggen) und Dinkel aus Plazenta-Zellen von speziell hergestellten oder handelsüblichen Plazenta-Zellinien der genannten Cerealen,
das gekennzeichnet ist durch die folgenden Stufen:

a) Aktivierung der gewählten Plazenta-Zelllinie einer der Cerealien aus der Gruppe Weizen, Reis, Gerste, Roggen, Hafer, Sorghum (Hirse), Triticale und Dinkel, in einem Wasserbad von vorzugsweise 25 bis 30 °C,

b) Zugabe eines vorgewärmten, für die jeweils gewählte Zelllinie vorgeschriebenen Kulturmediums unter Vermischung und anschließendes Inkubieren in an sich bekannter Weise, vorzugsweise bei etwa 37°C, und vorzugsweise in einer etwa 5 % $CO_2$ enthaltenden Atmosphäre,

c) Gewinnung der dabei gebildeten Zellen als Ausgangsmaterial für Großkulturen in Suspension durch Dekantieren des Nährmediums, Waschen der entstandenen Zellmasse mit einem geeigneten Puffer und Aufnahme der Zellen in einem kompletten Medium unter Einstellung einer für die Massenproduktion geeigneten Verdünnung,

d) Kultivierung der Zellen in Suspensionskultur im Großmaßstab auf an sich bekannte Weise durch Beimpfen einer Kultivierungsapparatur mit einer Zellsuspension in einem sterilen $CO_2$/Luft-Gemisch,

wobei durch Einbringen eines Plasmids und durch DNA-Analyse gezeigt werden kann, dass die in Masse gezüchteten Zellen denjenigen der Anfangs-Zellkultur entsprechen,

e) Herstellung eines Extrakts durch Behandlung des suspendierten geernteten Zellmaterials in einem organischen Lösungsmittel und/oder Wasser, Gewinnung der wäßrigen Phase vorzugsweise durch Zentrifugieren, Entfernung von restlichen Lipidanteilen (Rückstand I) aus der wäßrigen Phase, gegebenenfalls durch mehrmaliges Behandeln mit dem organischen Lösungsmittel unter Bildung eines wäßrigen Rohextrakts, gegebenenfalls in Form einer Suspension,

f) Reinigung bzw. Behandlung des Rohextrakts je nach Ausgangsmaterial; und

g) Sterilfiltration des Extrakts, vorzugsweise unter Verwendung eines 0,2 $\mu$m (Millipore®)-Filters oder einer PALL-Kerze, nach Einstellung des Extrakts auf den gewünschten pH-Wert, vorzugsweise 5,0 bis 7,2.

Die nach dem erfindungsgemäßen Verfahren erhältlichen Cerealien-Plazenta-Extrakte erreichen zumindest das biochemische Wirkungsprofil der bekannten natürlichen und synthetischen Cerealien-Plazenta-Extrakte, vorzugsweise weisen sie ein wesentlich verbessertes Wirkungsprofil auf, und sie sind insbesondere frei von unerwünschten Substanzen, speziell frei von immunogenen und pathogenen Proteinen und Protein-Abbauprodukten sowie deren unerwünschten Wirkungen, wie sie in natürlichen Cerealien-Plazenta-Extrakten vorkommen.

Die nach dem erfindungsgemäßen Verfahren erhältlichen Cerealien-Plazenta-Extrakte aus Plazenta-Zellen von Zelllinien der entsprechenden Cerealien eignen sich insbesondere zur Herstellung von kosmetischen Formulierungen, zur Herstellung von pharmazeutischen Präparaten und/oder zur Herstellung von Nahrungsergänzungsmitteln oder als Nahrungsergänzungsmittel selbst, die vorzugsweise (in verdünnter oder unverdünnter Form) in sie enthaltenden Kapseln zur direkten oralen Einnahme vorliegen können. Dadurch ist es möglich, die in der Pharmaindustrie, in der Kosmetikindustrie und in der Nahrungsmittellidustrie bekannten tierischen und pflanzlichen Plazenta-Extrakte, die sich durch eine Vielzahl von vorteilhaften inhalts- bzw. Wirkstoffen auszeichnen, die aber aufgrund ihres Gehaltes an Proteinen oder hochmolekularen Protein-Abbauprodukten, die pathogene und immunogene Eigenschaften aufweisen und bei regelmäßiger oder wiederholter Verabreichung - meistens verbunden mit lange Inkubationszeit beträgt - zu schwerwiegenden Erkrankungen führen, sogar carcinogen sein können und daher insbesondere im Zusammenhang mit TSE, speziell BSE (Rinderwahnsinn) und dem Jacob-Creutzfeld-Syndrom, weltweit in Verruf gekommen sind, teilweise oder vollständig zu ersetzen durch die erfindungsgemäß hergestellten, zuverlässig nebenwirkungsfreien Cerealien-Plazenta-Extrakte mit mindestens gleicher Wirksamkeit.

In dem weiter unten folgenden umfangreichen experimentellen Teil wurde anhand von einschlägigen Versuchen nachgewiesen, dass die erfindungsgemäß hergestellten Cerealien-Plazenta-Extrakte nicht nur frei von unerwünschten Nebenwirkungen sind (vgl. insbesondere die akute Toxizitätsprüfung, die kumulative Toxizitätsprüfung, die klinisch-toxikologische Prüfung nach intravenöser Verabreichung bei der Maus, der Sensibilisierungstest am Meerschweinchen, der Augenreiztest am Kaninchen, die klinisch-allergologische Prüfung im Patch-Test beim Menschen, die Mutagenitätsprüfung, die Prüfung auf Abwesenheit von Viren und Protonen), sondern auch vorteilhafte physiologische und metabolische Eigenschaften aufweisen (vgl. insbesondere die Verbesserung der Atmung im Warburg-Test, die Verbesserung des Wachstums im Guppy-Fisch- und Kaulquappen-Test, die Verbesserung der Glätte der Haut im modifizierten Padberg-Test, die Verbesserung der Wundheilung im Hautspannungstest, die Verbesserung des Feuchtigkeitsretentionsvermögens der Haut). Insbesondere die aus den nachfolgenden Ausführungsbeispielen ersichtlichen Stoffwechselaktivierungs- und Whitening-Effekte waren auch für den Fachmann auf diesem Gebiet durchaus überraschend.

Bevorzugte Ausführungsformen des erfindungsgemäßen Verfahrens sind solche, welche die folgenden Zusatzmerkmale, einzeln oder in Kombination, aufweisen, und dadurch gekennzeichnet sind, dass

eine in der Stufe (d) gewonnene pflanzliche Zellmasse in Ethylether oder Chloroform suspendiert und etwa 8 bis etwa 15 Tage lang bei etwa -10 bis etwa - 20°C im Kälteraum stehen gelassen und dann zur Gewinnung der wäßrigen Phase zentrifugiert wird;

die letzten Spuren von Ether oder Chloroform aus dem Dialysat (Außenlösung) in der Stufe (f) durch Stickstoff-Durchblasen bei pH 7,2 bis 7,5 entfernt werden;

als Zelllinie eine Plazenta-Zelllinie aus der Fruchtschale (Pericarp), der Samenschale (Testa) oder der proteinreichen Aleuronschicht von Cerealien aus der Gruppe Hafer, Gerste, Reis, Triticale, Roggen, Weizen, Dinkel und Sorghum

(Hirse) verwendet wird;

dann, wenn es auf das Zellwandmaterial ankommt, das in Wasser oder in einem organischen Lösungsmittel suspendierte Zellmaterial in der Stufe (e) in einem Ultraschallgenerator zerbrochen und das aufgeschlossene Material nach dem Zentrifugieren und Waschen mit Wasser durch chemische und/oder enzymatische Partialhydrolyse aufgeschlossen und dialysiert wird;

zur Herstellung von Pflanzen-Plazenta-Extrakten aus Zelllinien der Cerealien solche Ausgangsmaterialien verwendet werden, die keiner Genmanipulation unterzogen worden sind und die frei sind von unerwünschten Substanzen, speziell immunogenen und/oder pathogenen Proteinen (Prionen) und Proteinabbauprodukten, und in denen sich keine Insektizid-Rückstände nachweisen lassen;

ein oder mehrere von aus einer oder mehreren unterschiedlichen Cerealien-Plazenta-Zelllinlen gewonnenen, sterilfiltrierten Plazenta-Extrakten hergestellt und gegebenenfalls kombiniert wird (werden) mit einer oder mehreren aus einem oder mehreren unterschiedlichen tierischen Zelllinien gewonnenen, sterilfiltrierten tierischen Organextrakten sowie gegebenenfalls mit einem oder mehreren aus einem oder mehreren verschiedenen, synthetisch hergestellten tierischen Organextrakten und/oder mit einem oder mehreren verschiedenen, aus frischen tierischen Organen hergestellten tierischen Organextrakten und/oder gegebenenfalls mit einem oder mehreren aus einem oder mehreren verschiedenen, aus frischen Pflanzenorganen bzw. -teilen hergestellten Pflanzenextrakten;

wobei das Gewichtsverhältnis zwischen dem (den) pflanzlichen OrganExtrakt(en) und dem (den) tierischen Organ-Extrakt(en) (1 bis 99) : (99 bis 1), vorzugsweise (1 bis 50) : (50 bis 1), insbesondere (1 bis 20) : (99 bis 80), beträgt.

Gegenstand der Erfindung ist ferner gemäß einem weiteren Aspekt die Verwendung der Cerealien-Plazenta-Extrakte und Extraktgemische, die nach dem vorstehend beschriebenen erfindungsgemäßen Verfahren erhältlich sind, als Ersatz oder Ergänzung für pflanzliche und/oder tierische Organ-Extrakte, insbesondere Plazenta-Extrakte;

zur Herstellung eines Präparats mit Radikalfängerwirkung, insbesondere für kosmetische und/oder technische Zwecke;

zur Herstellung einer kosmetischen Formulierung;

zur Herstellung einer kosmetischen Formulierung mit die Melaninbildung hemmenden Eigenschaften (Whitening Effect);

zur Herstellung eines medizinischen Präparats für die topische, subkutane, intravenöse, intramuskuläre oder orale Applikation zum Zwecke der äußeren oder inneren Wundhellung, zur Behandlung von Arteriosklerose oder von Strahlungsschäden;

zur Herstellung eines medizinischen Präparats für die Stärkung des Immunsystems, für die Aktivierung des Zellstoffwechsels oder die Behandlung gastroenterologischer Erkrankungen, insbesondere Ulcera;

zur Herstellung eines Präparats mit aphrodisiakatischer Wirkung;

und der darin enthaltenen Rohextrakte und Vorstufen zur Stabilisierung und Haltbarmachung von gefärbten Geweben und Farbanstrichen, insbesondere Lackierungen;

zur Herstellung von Kulturlösungen, Nährlösungen und diätetischen Lösungen; und/oder

zur Herstellung von Nährlösungen und diätetischen Lösungen in sie (verdünnt oder unverdünnt) enthaltenden Kapseln für die direkte orale Einnahme, insbesondere als Nahrungsergänzungsmittel.

Zur Herstellung von Plazenta-Extrakten von spezifischen Cerealien nach dem erfindungsgemäßen Verfahren werden vorzugsweise die "Plazenten" der oben genannten Cerealien verwendet. Unter den "Cerealien-Plazenten" versteht man im vorliegenden Fall die Gesamtheit von Fruchtschale (Pericarp), Samenschale (Testa) und Aleuron-Schicht, nachstehend allgemein als "Fruchtschalen-Plazenta-Material" oder "Plazenta-Material" bezeichnet,

Die nach dem erfindungsgemäßen Verfahren erhältlichen Cerealien-Plazenta-Extrakte können auf zweierlei Weise hergestellt werden, nämlich aus Cerealien-Plazenta-Zelllinien, die aus frischem Cerealien-Plazenta-Material gewonnen worden sind, oder aus solchen, die in einer Zellbank hinterlegt sind. So sind beispielsweise in der Zellbank des pharmakologischen Instituts und in der Abteilung Biochemie des Chemischen Zentralinstituts der Brasilianischen Bundesuniversität Santa Maria (UFSM) S. Maria, RS, Brasilien, die folgenden, von Dr. A. Brown und Prof. Dr.Dr. R. Riemschneider hergestellten Zellkulturen von Secale cereale, Triticum vulgare, Oryza sativa, Triticale und anderen aufbewahrt und sie wurden dem Anmelder und den Haltern dieser Patentanmeldung auf Anforderung zur Verfügung gestellt. Die in der oben genannten Zellbank hinterlegten pflanzlichen Plazenta-Zelllinien sind frei zugänglich und können von beliebigen Dritten von der oben genannten Universität bezogen werden.

Die aus Cerealien-Plazenta-Zelllinien gewonnenen, sterilfiltrierten erfindungsgemäß hergestellten Cerealien-Plazenta-Extrakte können einzeln oder im Gemisch, vorzugsweise im Gemisch mit mindestens einem synthetisch hergestellten Pflanzen-Plazenta-Extrakt oder mit mindestens einem aus frischem pflanzlichem Plazenta-Material hergestellten Pflanzen-Plazenta-Extrakt oder mit beiden kombiniert werden, wobei das Gewichts-Mengenverhältnis zwischen dem aus den pflanzlichen Plazenta-Zelllinien gewonnenen, sterilfiltrierten Plazenta-Extrakt und dem synthetisch hergestellten Pflanzen-Plazenta-Extrakt und/oder dem aus frischen Pflanzen-Plazenta-Materialien hergestellten Pflanzen-Extrakt vorzugsweise (1 bis 99):(99 bis 1), insbesondere (1 bis 50):(50 bis 1), speziell (1 bis 20):(99 bis 80), beträgt.

Das erfindungsgemäße Verfahren zur Herstellung von Cerealien-Plazenta-Extrakten, insbesondere von Cerealien aus der Gruppe Weizen, Reis, Mais, Gerste, Roggen, Hafer, Sorghum (Hirse), Triticale (eine Kreuzung aus Weizen und Roggen) und Dinkel, wird im Einzelnen wie folgt durchgeführt:

I) In einer ersten Stufe wird ein frisch hergestelltes oder handelsübliches Cerealien-Plazenta-Zelllinienmaterial in einem Wasserbad von vorzugsweise 25 bis 30 °C aktiviert, bevor nach der Zugabe eines vorgewärmten, für die jeweils gewählte Zelllinie vorgeschriebenen Kulturmediums durch Vermischen und anschließendes Inkubieren in an sich bekannter Welse, vorzugsweise bei etwa 37 °C und vorzugsweise in einer etwa 5 % $CO_2$ enthaltenden Atmosphäre, die dabei gewonnenen Zellen als Ausgangsmaterial für Großkulturen in Suspension durch Dekantieren des Nährmediums, Waschen der entstandenen Zellmassen mit einem geeigneten Puffer und Aufnahme der Zellen in einem kompletten Medium unter Einstellung einer für die Massenproduktion geeigneten Verdünnung im Großmaßstab in Suspensionskultur auf an sich bekannte Weise kultiviert werden durch Beimpfung der Kultivierungsapparatur mit einer Zellsuspension in einem sterilen $CO_2$/Luft-Gemisch zur Bildung der gewünschten Zellmasse, wobei durch Einbringen eines Plasmids und durch DNA-Analyse gezeigt werden kann, dass die in Masse gezüchteten Zellen denjenigen der Anfangs-Zellkultur entsprechen.

II. Danach wird in einer zweiten Stufe das in der Stufe (I) gewonnene suspendierte Zellmaterial zur Herstellung eines Extrakts in einem organischen Lösungsmittel und/oder Wasser behandelt,
die wässrige Phase wird vorzugsweise durch Zentrifugieren gewonnen und von restlichen Lipidanteilen (Rückstand I) befreit, gegebenenfalls durch mehrmaliges Behandeln mit dem organischen Lösungsmittel unter Bildung eines wässrigen Rohextrakts, gegebenenfalls in Form einer Suspension,
der Rohextrakt wird je nach Ausgangsmaterial durch Dialyse gereinigt bzw. behandelt und
der dabei erhaltene Extrakt wird sterilfiltriert, vorzugsweise unter Verwendung eines 0,2 $\mu$m Millipore®-Fifters oder einer Pall-Kerze, nach Einstellung des Extrakts auf den gewünschten pH-Wert von vorzugsweise 5,0 bis 7,2.

Allgemein kann das erfindungsgemäße Verfahren zur Herstellung der Cerealien-Plazenta-Extrakte durch das folgende Reaktionsschema, dargestellt am Beispiel der Herstellung eines Plazenta-Extrakts von Secale cereale, wie folgt veranschaulicht werden:

Ausgangsmaterial: Fruchtschalen-Plazenta-Zelllinien-Material von Secale cereale (Carpel) aus Roggen definierter Herkunft, genetisch unbehandelt, ohne Insektizidrückstände

Anlegen einer Zellkultur aus Fruchtschalen-Plazenten (Zelllinienkultur) und ggf. Hinterlegen der Zellkultur bei einer Datenbank

↓

Aktivieren eines handelsüblichen Pflanzen-Plazenta- Zelllinlenmaterials im Wasserbad
Zugabe eines vorgewärmten für die jeweils gewählten Zellen vorgeschriebenen Kulturmediums unter Vermischen und anschließdem Inkubieren in an sich bekannter Weise (37 °C/5 % $CO_2$/Luft),
Gewinnung der gebildeten Zellen als Ausgangsmaterial für Großkulturen in Suspension durch Dekantieren des Nährmediums, Waschen der entstandenen Zellmasse mit einem geeigneten Puffer und Aufnahme der Zellen in einem kompletten Medium und Einstellung einer für die Massenproduktion geeigneten Verdünnung,
Kultivieren der Zellen in Suspenslonskultur im Großmaßstab auf an sich bekannte Weise durch Beimpfen der Kultivierungsapparatur mit einer Zellsuspension in einem sterilen $CO_2$/Luft-Gemisch

↓

Fortsetzung des Verfahrens nach
der weiter unten folgenden
Übersicht zu 1.1 oder 1.2 (s. Tafeln I und II auf S. 30 und 32)

Die erfindungsgemäß hergestellten wasserlöslichen Cerealien-Plazenta-Extrakte lassen sich sowohl mit synthetischen pflanzlichen und/oder tierischen Organextrakten, wie sie in EP-A-0 552 516 beschrieben sind, als auch mit natürlichen pflanzlichen und/oder tierischen Organextrakten oder beiden kombinieren. Auch im Falle dieser Kombinationen werden verbesserte biochemische Wirkungsprofile erzielt, die besser sind als diejenigen der synthetischen Organextrakte allein, und die frei sind von den unerwünschten Nebenwirkungen der natürlichen Organextrakte. Erfindungsgemäß ist es insbesondere möglich, auf technisch einfache und besonders elegante Weise garantiert virusfreie und prionenfreie Kombinationen von synthetischen Organextrakten und pflanzlichen Zelllinien-Organextrakten herzustellen, was besonders bedeutsam ist im Zusammenhang mit den heute sehr akuten TSE-Problemen, speziell BSE.

Vorzugsweise werden die erfindungsgemäß hergestellten Cerealien-Plazenta-Extrakte aus einer im Handel erhältlichen Cerealien-Plazenta-Zelllinie der jeweiligen Cerealle nach einem Verfahren hergestellt, das durch die folgenden Stufen gekennzeichnet ist:

a) Aktivierung der Zellkultur der gewählten pflanzlichen Zellen in einem Wasserbad von vorzugsweise 25 bis 30°C,
b) Zugabe eines vorgewärmten, für die jeweils gewählten Zellen vorgeschriebenen Kulturmediums unter Vermischung und anschließendes inkubieren in an sich bekannter Weise, vorzugsweise bei etwa 37°C und vorzugsweise in einer etwa 5 % $CO_2$ enthaltenden Atmosphäre,
c) Gewinnung der dabei gebildeten Zellen als Ausgangsmaterial für Großkulturen in Suspension oder auf Oberflächen oder in Suspension durch Dekantieren des Nährmediums, Waschen der entstandenen Zellmasse mit einem geeigneten Puffer und Aufnahme der Zellen in einem kompletten Medium unter Einstellung einer für die Massenproduktion geeigneten Verdünnung,
d) Kultivierung der Zellen in Suspensionskultur im Großmaßstab auf an sich bekannte Weise durch Beimpfen der Kultivierungsapparatur mit einer Zellsuspension in einem sterilen $CO_2$/Luft-Gemisch, zur Bildung der gewünschten Zellmasse, wobei durch Einbringen eines Plasmids und durch DNA-Analyse gezeigt werden kann, dass die in Masse gezüchteten Zellen denjenigen der Anfangs-Zellkultur entsprechen,
e) Herstellung eines Extrakts durch Behandlung des suspendierten geernteten Zellmaterials in einem organischen Lösungsmittel und/oder Wasser, Gewinnung der wäßrigen Phase vorzugsweise durch Zentrifugieren (Trommelzentrlfuge), Entfernung von restlichen Lipidanteilen aus der wäßrigen Phase durch mehrmaliges Behandeln mit dem organischen Lösungsmittel unter Bildung eines wäßrigen Rohextrakts, gegebenenfalls in Form einer Suspension,
wobei zur Gewinnung der öllöslichen Anteile eine Extraktion des Trommelzentrifugen-Rückstandes mit Olivenöl/Sonnenblumenöl/lsopropylmyristat bzw. eine Extraktion von getrocknetem geerntetem Zellmaterial direkt mit einem Lipoidgemisch wie angegeben oder ähnlich erfolgt,
f) weitere Reinigung bzw. Behandlung der Rohextrakte je nach Material und
g) Sterilfiltration der Extrakte, vorzugsweise unter Verwendung eines 0,2 bzw. 0,1 $\mu$m Millipore®-Filters oder einer PALL-Kerze nach Einstellung des Extrakts auf den gewünschten pH-Wert, vorzugsweise 5,0 bis 7,2.

Bevorzugte Ausführungsformen des erfindungsgemäßen Verfahrens zur Herstellung von Cerealien-Plazenta-Extrakten sind dadurch charakterisiert, dass
eine in der Stufe (d) gewonnene pflanzliche Zellmasse in Ethylether oder Chloroform suspendiert und etwa 8 bis etwa 15 Tage lang bei etwa -10 bis etwa -20°C im Kälteraum stehen gelassen und dann zur Gewinnung der wäßrigen Phase zentrifugiert wird;
die letzten Spuren von Ether oder Chloroform aus dem Dialysat (Außenlösung) in der Stufe (f) durch Stickstoff-Durchblasen bei pH 7,2 bis 7,5 entfernt werden; und/oder
In der Stufe (a) Zellkulturen aus handelsüblichen pflanzlichen Zelllinien einschließlich der in der Beschreibung genannten nicht-handelsüblichen speziellen pflanzlichen Zeillinien einzeln oder im Gemisch eingesetzt werden.

Zur Herstellung der erfindungsgemäß hergestellten Cerealien-Plazenta-Extrakte und zur Durchführung des erfindungsgemäßen Verfahrens werden vorzugsweise als Plazenta-Zelllinien mindestens eine der "Plazenta-Zeillinien", der Cerealien aus der Gruppe Gerste, Hafer, Reis, Triticale, Roggen, Weizen, Dinkel, und Sorghum (Hirse) verwendet.

In der Zellbank des Pharmakologischen Instituts und in der Abteilung Biochemie des Chemischen Zentralinstituts der Brasillanischen Bundesuniversität Santa Maria (UFSM) S. Maria, RS, Brasilien, werden die folgenden, von Dr. A. Brown und Prof. Dr.Dr. R. Riemschneider hergestellten Zellkulturen von Secale cereale (Roggen), Triticum vulgare, Oryza sativa, Triticale und andere aufbewahrt und sind dem Anmelder und den Haltern dieser Patentanmeldung auf Anforderung zur Verfügung gestellt worden. Sie können von bellebigen Dritten von der genannten Universität bezogen werden.

Bei der Herstellung der Cerealien-Plazenta-Extrakte wird in der Stufe (e) die wässrige Phase des längere Zeit mit einem oder mehreren organischen Lösungsmitteln behandelten Zellmaterials alkalisch und/oder sauer und/oder mit organischen Lösungsmittel weiter behandelt, gegebenenfalls auch in einem Ultraschallgenerator "zerbrochen", und das aufgeschlossene Material wird nach dem Zentrifugieren und Waschen mit Wasser durch chemische und/oder enzymatische Partialhydrolyse aufgeschlossen und gegebenenfalls anschlie-ßend dialysiert.

Weitere bevorzugte Ausgestaltungen des erfindungsgemäßen Verfahrens bestehen darin, dass zur Herstellung von Pflanzen-Organextrakten aus Plazenta-Zelllinien von Cerealien solche Ausgangsmaterialien verwendet werden, die keiner Genmanipulation unterzogen worden sind und die frei sind von unerwünschten Substanzen, speziell immunogenen und/oder pathogenen Proteinen (Prionen) und Proteinabbauprodukten, wie sie in natürlichen Pflanzenextrakten mit unerwünschten Wirkungen zu finden sind, und in denen sich keine Insektizid-Rückstände nachweisen lassen.

Gemäß einem weiteren Aspekt betrifft die vorliegende Erfindung die Verwendung der vorstehend beschriebenen wäßrigen Cerealien-Plazenta-Extrakte, einzeln oder in Form von Gemischen oder Kombinationen derselben, als Ersatz oder Ergänzung für Plazenta-, Kollagen- oder andere Organextrakte bzw. Kombinationen davon sowie zur Herstellung von kosmetischen Formulierungen und zur Herstellung medizinischer Präparate für die Stärkung des Immunsystems, für die Aktivierung des Zellstoffwechseis oder für die Behandlung gastroenterologischer Erkrankungen, insbesondere Ulcera, und für die topische, subkutane, intravenöse oder Intramuskuläre Applikation zum Zwecke der äußeren oder Inneren Wundheilung, zur Behandlung von Arteriosklerose oder von Strahlungsschäden.

Die vorstehend beschriebenen erfindungsgemäßen wäßrigen Pflanzenorganextrakte und Extrakt-Kombinationen eignen sich auch zur Herstellung von Präparaten mit aphrodisiakatischer Wirkung, Dies gilt insbesondere für die aus pflanzlichen Cerealien-Zelllinien gewonnenen Extrakte in Kombination mit vorzugsweise aus Ginkgo biloba, Sycamore, Saccharomyces, Torula oder Cyanophyten gewonnenen Extrakten und ihren Kombinationen mit auf tierischen Zelllinien basierenden Extrakten, die als Wirkstoffe in Präparaten mit aphrodisiakatischer Wirkung verwendet werden können. Die Cerealien-Plazenta-Extrakte können auch - einzeln oder in Form von Gemischen oder Komblnatlonen, unverdünnt oder verdünnt - in Form von sie enthaltenden Kapseln für die direkte orale Einnahme, beispielsweise als Nahrungsergänzungsmittel, verwendet werden.

Außerdem betrifft die vorliegende Erfindung die Verwendung der vorstehend beschriebenen Pflanzenextrakte und Extraktgemische einschließlich der Rohextrakte, Rohextraktgemische und ihrer Vorstufen zur Stabilisierung und Haltbarmachung von gefärbten Geweben, Farbanstrichen und Lackierungen, insbesondere solchen mit Lacken auf Wasserbasis, sowie zur Herstellung von Kulturlösungen, Nährlösungen und diätetischen Lösungen.

Das erfindungsgemäße Verfahren, insbesondere die einzelnen Stufen (a) bis (g) des erfindungsgemäßen Verfahrens, werden nachstehend unter Bezugnahme auf die beiliegenden Zeichnungen näher erläutert.

ad Stufe (a)

Auftauen von tiefgefrorenen pflanzlichen Zellkulturen aus kompetenten Quellen, aus eigener Zucht oder Zelllinien, wie sie im Handel sind, von Zelllinien der Cerealien aus der Gruppe Hafer, Gerste, Reis, Triticale, Roggen, Weizen, Dinkel und Sorghum (Hirse);

ad Stufe (b)

Inkubieren der jeweiligen pflanzlichen Zellkulturen nach den gegebenen Vorschriften;

ad Stufe (c)

Gewinnung von pflanzlichen Zellen als Ausgangsmaterial für Großkulturen;

ad Stufe (d)

Kultivierung der pflanzlichen Zellen in Suspension zunächst in Suspensionskulturflaschen (Figur 1 und Figur 2), dann in Rohrschlangen-Kultivatoren IGV von 50 L bis 2000 L gemäß Figur 3;

<u>ad Stufe (e)</u>

<u>Extraktion</u> durch Behandlung des suspendierten geernteten pflanzlichen Zellmaterials bei 0°C, vorzugsweise bei -10 °C bis -20 °C, in organischen Lösungsmitteln für vorzugsweise 6 bis 14 Tage;

bzw. durch Gewinnung eines wässrigen Rohextrakts vorzugsweise durch Zentrifugieren mit beispielsweise 3000 UpM, wobei zuweilen der Zentrifugierenrückstand wichtig ist für die öllöslichen Extrakte;

<u>ad Stufen (f) und (g)</u>

<u>weitere Verarbeitung</u> zu reinen, Protein-haltigen oder Protein-freien Pflanzen-Plazenta-Extrakten, die vor ihrer Lagerung sterilfiltriert werden.

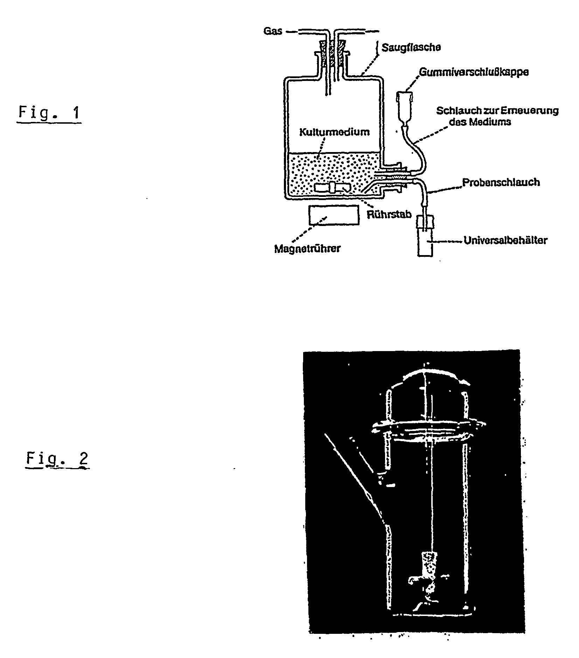

Für den Fall, dass die gewählte pflanzliche Zelllinie in Suspensionskultur wächst, wird mit der "Rotary Shaker Technique" begonnen und dann wird in Suspensionskulturflaschen, z.B. gemäß beiliegenden Figuren 1 und 2, weiter gearbeitet.

Die Suspensionskulturen in sehr großem Maßstab werden dann in 50 L- bis zu 1500 L-Fermentern, z.B. nach Moore et al., durchgeführt.

Bewährt haben sich auch Rohrschlangen-Kultivatoren IGV von 50L bis zu 2000L zur Biomasse-Produktion pflanzlicher Zellen gemäß beiliegender Fig. 3.

Die Erfindung wird nachstehend unter Bezugnahme auf die beiliegenden Zeichnungen näher erläutert. Es zeigen:

FIg. 1     eine Suspensionskulturflasche, die aus einer Saugflasche hergestellt ist. Um zwischendurch Kulturmedium zugeben und Proben entnehmen zu können, sind der Schlauch zur Erneuerung des Kulturmediums und der Probenschlauch mit einer Klemme abgequetscht. Zum "Futtern" wird die Klemme vom Schlauch zur Erneuerung des Kulturmediums entfernt und Kulturmedium wird durch die Gummiverschlußkappe injiziert. Zur Proben-Entnahme wird die Klemme vom Probenschlauch entfernt. Danach wird der Universalbehälter durch einen sterilen ersetzt. Das Gefäß sollte nicht mehr als zu einem Viertel gefüllt sein. Es sei darauf hingewiesen, dass der "Kragen" um den Magnetstab herum dazu dient, den Kontakt zwischen dem Magneten und dem Boden der Flasche möglichst klein zu halten;

Fig. 2     ein Gefäß, das zur Züchtung von Zellen in Suspension dient. Der mit Kunststoff überzogene Magnet wird durch einen außerhalb des Gefäßes befindlichen Magnetrührer gedreht; und

Fig. 3     einen Rohrschlangen-Kultivator IGV zur Produktion von pflanzlichen Zellen in Suspensions-Kulturen.

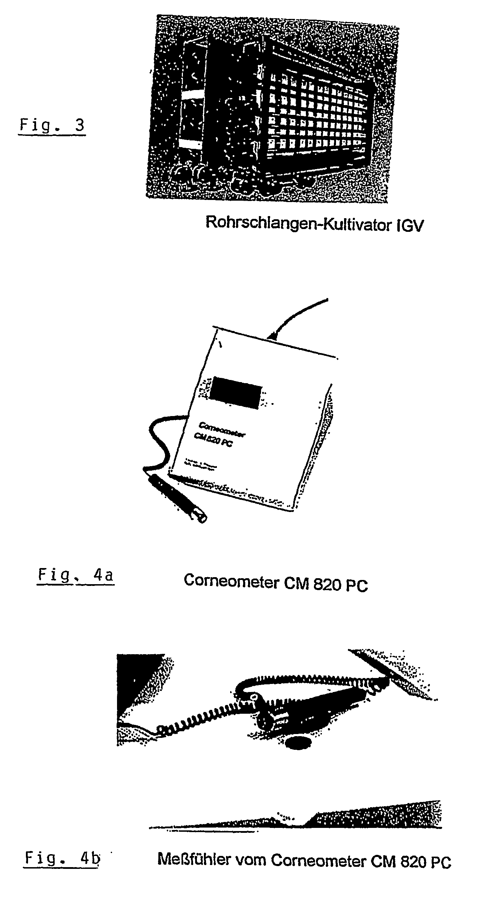

Fig. 4a     eine Vorrichtung zur Messung der Hautfeuchtigkeit (Corneometer CM 820 PC);

Fig. 4b     den Meßfühler eines Corneometers CM 820 PC mit einem flachen Kondensator als Stirnfläche, der auf die Haut gedrückt wird zur Messung der Hautfeuchtigkeit;

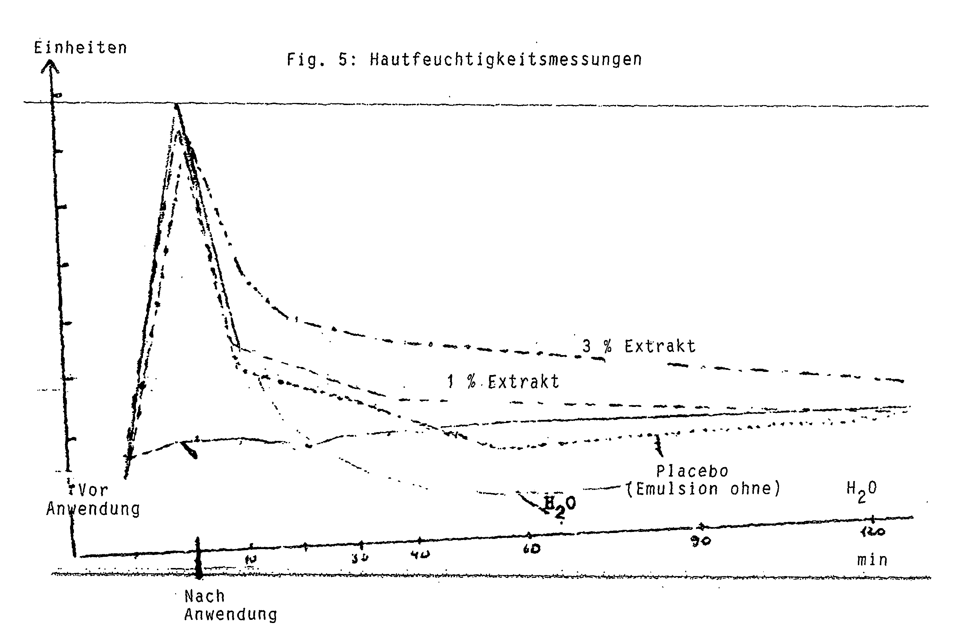

Fig. 5     eine graphische Darstellung des Feuchtigkeitshaltevermögens der Haut (ln h) nach einmaliger Applikation einer Hautcreme ohne Zusätze (Kontrolle) bzw. einer Hautcreme mit zugesetztem Triticale-"Plazenta"-Extrakt in unterschiedlichen Mengen (1 % und 3 %) gemäß dem weiter unten folgenden Beispiel 5.

Die Herstellung der Organextrakte aus den "Plazenta"-Anteilen der oben genannten Cerealien via Zelllinien-Kultur verläuft im Wesentlichen in drei Stufen, die nachstehend kurz beschrieben werden:

1) Begonnen wird mit dem Anlegen einer bzw. von zwei "Plazenta"-Zellkulturen aus Zellen der Fruchtblätter der Samenleisten und der proteinreichen Aleuronschicht von Cerealien, die nicht genmanipuliert worden sind und in denen sich keine Insektizid-Rückstände nachweisen lassen.

Lege artis sind seinerzeit den Fruchtblättern der Cerealien-Samenanlage und der Cerealien-Aleuronschicht Zellen entnommen und zum Anlegen von Kulturen in geeigneten Nährlösungen gezüchtet worden. Die beiden Zellkulturen wurden unter geeigneten Bedingungen tiefgefroren und in der Bundesuniversität Santa Maria (UFSM), S. Maria, RS, Brasilien, aufbewahrt. Angaben über die Zusammensetzung der hierfür erforderlichen Nährlösungen sowie auch der Lösungen der nachfolgenden Stufe (2) finden sich in den weiter unten folgenden Beispielen.

2) Gewinnung einer ausreichenden Menge Pflanzen-Zellmasse aus den beiden unter (1) angelegten Cerealien-"Plazenta"-Zellkutluren, zunächst in einer Anzahl von Suspensionskultur-Flaschen von jede 10 L, dann in Fermentern (1000 bis 4000 L) bzw. Rohrschlangen-Kultivatoren (1000 bis 2000 L).

3) Herstellung des Cereallen-"Plazenta"-Extrakts aus der unter (2) gewonnenen Zellmasse (siehe die weiter unten folgenden Beispiele).

In allen Fällen ist es wichtig, dass sterile Bedingungen streng eingehalten werden und zwar sowohl hinsichtlich der Apparaturen als auch hinsichtlich der in beträchtlichen Mengen erforderlichen, vorher zu testenden und zu sterilisierenden Kulturmedien.

Hinsichtlich der Zellvermehrung kann aufgrund arithmetischer Überlegungen erwartet werden, dass die beschriebenen Großkultur-Verfahren innerhalb verhältnismäßig kurzer Zeiten ausreichende Zellmaterial-Mengen, ausgehend von einer gefrorenen Zellkultur, liefern werden. Langsam wachsende Zellen vermehren sich in einer Woche auf das 10-fache, in 6 Wochen auf das $10^6$-fache. Aus einer Zelle können innerhalb von 12 Wochen $10^{12}$ Zellen (4 kg), innerhalb von 15 Wochen $10^{16}$ Zellen (4000 kg) entstehen.

Bei der Entwicklung und beim Einsatz von geeigneten Nährlösungen ist darauf zu achten, dass nur Komponenten zum Einsatz kommen, die in dem angestrebten Organextrakt später keine pharmakologisch-toxikologischen und dermatologisch unerwünschten Effekte verursachen. Dies gilt vor allem im Falle einer medizinischen, kosmetischen und diätetischen Anwendung der erfindungsgemäß hergestellten Pflanzen-Plazenta-Extrakte.

Die Auswahl geeigneter Nährlösungen und die Standardisierung der Bedingungen bei der Zellvermehrung zur Gewinnung einer Zellmasse sind wichtig, um die Zelllinien bei der Vermehrung vor Veränderungen und Mutationen zu bewahren. Durch Einbringen eines Plasmids und durch DNA-Analyse kann gezeigt werden, dass die in Masse gezüchteten Zellen denjenigen der Ausgangs-Zellkultur entsprechen.

Die erfindungsgemäß hergestellten wasserlöslichen Cerealien-Plazenta-Extrakte, lassen sich sowohl mit synthetischen pflanzlichen und/oder tierischen Organextrakten wie sie in EP-A-0 552 516 beschrieben sind, als auch mit natürlichen pflanzlichen und/oder tierischen Organextrakten oder beiden kombinieren. Auch im Falle dieser Kombinationen werden verbesserte biochemische Wirkungsprofile erzielt, die besser sind als diejenigen der synthetischen Organextrakte und die frei sind von den unerwünschten Nebenwirkungen der natürlichen Organextrakte. Die Erfindung erlaubt insbesondere eine technisch einfache und besonders elegante Herstellung von garantiert virusfreien und prionenfreien Organextrakten, die eine Kombination von synthetischen Extrakten und Zelllinien-Extrakten darstellen, was besonders bedeutsam ist im Zusammenhang mit den TSE-Problemen, speziell BSE.

Die erfindungsgemäß hergestellten Cerealien-Plazenta-Extrakte enthalten die Cereallen-Plazenta in der Regel in verdünnter Form mit einem Plazenta-Trockensubstanzgehalt von 0,001 bis 15 Gew.-%, vorzugsweise von 0,1 bis 10 Gew.-%, insbesondere von 1 bis 6 Gew.-%.

Die Cerealien-Plazenta-Extrakte selbst werden bei den oben genannten Verwendungen in Mengen von 0,001 bis 20 Gew.-%, vorzugsweise von 0,1 bis 12 Gew.-%, insbesondere von 0,5 bis 6 Gew.-%, eingesetzt.

Die Gewichts-Mengenverhältnisse, die bei der Kombination von Zellextrakten aus pflanzlichen und tierischen Zelllinien mit entsprechenden synthetischen Zellextrakten anzuwenden sind, können in weiten Bereichen variiert werden, z.B. zwischen (1 bis 50):(98 bis 50), vorzugsweise (1 bis 20):(99-80), insbesondere (1 bis 10):(99 bis 90), liegen. Entsprechendes gilt auch für die Extrakt-Kombination, die aus frischen Pflanzenorganen und zugehörigen pflanzlichen Zelllinien hergestellt werden. Auch Kombinationen aller drei Extrakt-Arten sind von interesse, und zwar in vielen Bereichen von beispielsweise (1 bis 20):(1 bis 10):(Rest synthetischer Extrakt), vorzugsweise (1 bis 10):(1 bis 5):(Rest synthetischer Extrakt), insbesondere (1 bis 5):(1 bis 5):(Rest synthetischer Extrakt), für Zelllinien-Organextrakt zu entsprechendem natürlichem Organextrakt zu entsprechendem synthetischem Organextrakt. Die angegebenen Mengenverhältnisse sollen die Erfindung besser verständlich machen, die Erfindung ist darauf aber nicht beschränkt.

Wie in der mehrfach zitierten EP-A-0 552 516 ausgeführt, lassen sich durch Erhöhung der Anteile an bestimmten Komponenten, z.B. an Glycin bis auf 4,5 %, durch Erhöhung der Anteile an Sorbit, Mannit bis auf 6 % und durch Erhöhung der Anteile an Panthenol bis auf 35 %, besondere kosmetische Effekte erzielen, die Naturextrakten fehlen.

Die erfindungsgemäß hergestellten Cerealien-Plazenta-Extrakte, werden üblicherweise durch die folgenden Kennzahlen charakterisiert:

pH-Wert, Trockenrückstand (5 h bei 105°C), N-Gehalt (nach Kjeldahl), Aminosäurebestimmung: qualitativ mittels Ninhydrin, mittels Dünnschichtchromatographie (TLC) und HPLC; Peptidnachweis: mittels Biuret-Reagens, Protein-Freihelt (mittels Sulfosalicylsäure); Nucleinsäurekomponenten: TLC. Kohlenhydrat-Komponenten: TLC, UV-Spektrum (1:50 oder 1:100), HPLC, Sterilitätsprüfungen nach DAB 10.

Besondere Kennzahlen, die zur Charakterisierung der Plazenta-Extrakte von Cereallen herangezogen werden können, sind vor allem Messungen zur Bestimmung der Stoffwechsel-Aktivitäten, wie z.B. der Warburg-Versuch zur Bestimmung von Atmungs- und Gärungssteigerungen und Wachstumssteigerungen bei Fischen, Kaulquappen: Metamorphosebeschieunigung.

Die Wirkungsweise aller obengenannten Extrakte wurde mit Hilfe folgender Untersuchungen verglichen und gestützt (biochemische und biologische Tests):

Tabelle I

Methoden zur Prüfung der Wirkung von Organextrakten

| Nr. | Methode (beschrieben in DE 196 24 476) |
|---|---|
| 1 | Messung der Steigerung der Gewebsatmung z.B. von Leberhomogenat einer Ratte oder eines Meerschweinchens im Warburg-Test zur Bestimmung des **Atmungssteigerungsfaktors** (die Atmung des Leberhomogentas wird auf 1,0 festgesetzt): Faktorangabe |
| 2 | **Wachstumstest** zur Beurteilung der Steigerung der Stoffwechselaktivität beispielsweise durch Messung des Wachstums von |
| | a) Guppy-Fischen durch Längenmessung in mm nach 20 Tagen [Tp 80-100], (Kontrollwerte in runden Klammern) |
| | b) Kaulquappen von **Xenopus laevie** DAUDIN durch Längenmessung, Gewichtszunahme und Vorverlegung der Metamorphose (Beginn und Ende bei der Umwandlung zum Frosch) in Tagen |
| 3 | Messung der Hautglättung nach der modifizierten **PADBERG-Methode** unter Verwendung von 3 %igen WIÖ-Emulslonen nach 14-tägiger Anwendung; Messung der Photometerwerte in % von "behandelt" im Vergleich zum Ausgangszustand (Kontrollwerte in Klammern) |
| 4 | Profillinlen-Messung nach der **Bildanalyse;** beurteilt werden die Profillinienflächen FA und die Linienlänge LL |
| 5 | Messung des **Wasserrückhaltevermögens** mittels der Haut eines Corneometers |
| 6 | Patch-Test am Menschen, d.h. **allergologische** Prüfung |
| 7 | Messung der Steigerung der **Hautspannung** im Wundheilungstest nach 21 Tagen ($\Delta$-Werte) |

Im Falle medizinischer Anwendung der Organextrakte ist Prüfung auf Pyrogene im Kaninchen- bzw. LAL-Test erforderlich. Besondere Kennzahlen, die zur Charakterisierung der Extrakte herangezogen werden, sind die Hydroxyprolin-Bestimmung z.B. für pflanzliche Extrakte, die aus Zellwandmaterial gewonnen werden können. Manche Hefezell-Präparationen lassen sich durch ihre hohe Gärungssteigerungs-Aktivität im WARBURG-Versuch charakterisieren. Entsprechendes gilt auch für die Atmungssteigerung solcher Präparate (vgl. DE-C$_2$-40 42 157 (Salvioni)).

Die dermatologische Verträglichkelt und die Toxikologie wurden in üblicher Weise nach den in der folgenden Tabelle II angegebenen Methoden geprüft:

Tabelle II

Akute Toxizitätsprüfung nach intravenöser Applikation an der Maus.

Kumulative Toxizitätsprüfung nach 7-täglger peroraler Applikation an der Maus.

Kilnisch-toxikologische Beobachtungen nach intravenöser Verabreichung bei der Maus (Screening nach R.T. Turner): 2 g/kg, 10 g/kg, 20g/kg Maus.

Offener epikutaner Sensibilisierungstest am Meerschweinchen über 3 Wochen (nach Bühler).

Augenreiztest am Kaninchen in Anlehnung an "Appraisal of the Safety of Chemicals in Foods, Drugs and Cosmetics" von The Staff of Division of Pharmacology, FDA, Bewertung von Augen-Läsionen nach Draize, 1959.

Prüfung auf primäre Hautreizung beim Kaninchen in Anlehnung an "Appraisal of the Safety of Chemicals in Foods, Drugs and Cosmetics" von The Staff of Division of Pharmacology, FAD, Hautglftigkeit nach Draize, 1959.

Klinisch-allergologische Prüfung Im Patch-Test beim Menschen.

Ames-Test: Zur Prüfung auf mutagene Wirkung wird der Ames-Test ohne und mit metabolischer Aktivierung herangezogen: Mutagenitätsprüfung (Screening).

Abwesenheit von Viren und Prionen: Sie kann durch den Einsatz von lege artis hergestellten Zellkulturen und von Zellkulturen aus kompetenten Quellen garantiert werden. Die in der Beschreibungseinleitung weiter oben erwähnten Probleme mit frischem Pflanzen und Organmaterial entfallen erfindungsgemäß vollkommen.

Die lege artis, d.h. nach GMP (Good Manufacturing Practice), erfindungsgemäß hergestellten Cerealien-Plazenta-Extrakte passieren sämtliche genannten Tests problemlos - sachgemäße Produktion vorausgesetzt. Weitere Angaben sind in den weiter unten folgenden Beispielen enthalten.

Die Erfindung wird durch die folgenden Beispiele näher erläutert, ohne jedoch darauf beschränkt zu sein.

Beispiel 1

Herstellung eines Roggen-"Plazenta"-Extrakts (Roggen = Cereale secale)

1.1 Zunächst erfolgt die Aktivierung der beiden "Plazenta"-Zellkulturen aus Roggen-Fruchtschalen der Samenanlagen sowie aus der proteinreichen Aleuronschicht von Roggenkörnern durch Mischen mit dem vorgewärmten Kulturmedium (s. unten), anschließendes Inkubieren bei 37 °C in einer 5 % $CO_2$ enthaltenden Atmosphäre, Als Kulturmedium wurde das folgende verwendet (Angaben in g/L):

| | |
|---|---|
| $MgSO_4.7H_2O$ | 0,15 - 0,3 |
| $Ca(NO_3)_2$ | 0,2 - 0,4 |
| $KNO_3$ | 0,05 - 0,09 |
| KCl | 0,04 - 0,09 |
| $Na_2SO_4$ | 0,1 - 0,4 |
| $MnSO_4.4H_2O$ | 0,005 - 0,01 |
| $ZnSO_4.7H_2O$ | 0,001 - 0,006 |
| KJ | 0,001 - 0,003 |
| $Fe_2(SO_4)_3.6H_2O$ | 0,001 - 0,004 |
| $NaH_2PO_4.H_2O$ | 0,04 - 0,09 |
| Ca-Pantothenat | 0,00008 - 0,00015 |
| Thiamin . HCl | 0,00008 - 0,00016 |
| Glycin | 0,002 - 0,009 |
| Methionin | 0,0001 - 0,0003 |
| Asparagin | 0,0001 - 0,0008 |
| Cystein . HCl | 0,008 - 0,0022 |
| Saccharose | 20 g und |
| Kokosnussmilch | 20 % (deproteinisiert: 15 min , 15 at und sterilisiert) |

Die dabei gebildeten Zellen, die als Ausgangsmaterial für eine Großkultur in Suspension verwendet werden, werden durch Dekantieren des ersten Nährmediums, Waschen der entstandenen Zellmasse mit einem geeigneten Puffer und Aufnahme der Zellen in einem kompletten Medium unter Einstellung einer für die Massenproduktion geeigneten Verdünnung gewonnen.

Tafel I: Übersicht zu 1.1

**1.1. Zellmasse aus Fruchtschalen-Plazenta + Zellmasse aus Aleuronschicht
(Carpel von Secale cereale)**

Vereinigen und mit Ethylether bei -20 °C behandelt

↓

Trommelzentrifuge

Lösung I          Rückstand I

NaOH / \ HCl          ↓

Extraktlösung II     Extraktlösung III          Beispiel 2  →

↓

pH-Einstellung auf 6,7

↓

Filtration

Rückstand II     Lösung IV

↓

Behandlung mit Butylenglycol/Ethanol/Wasser

↓

Abziehen der Lösungsmittel im Vakuum 0,1 Torr

↓

fl. Rückstand

Vereinigen mit Lösung IV

↓

Rückstand          Filtrat: Lösung V

Lösung V wird analysiert und auf den gewünschten Standard-pH-Wert 6,7 eingestellt (Standardisierung), z.B.: pH 6,7; Trockenrückstand 1,6 - 1,8 %; N auf 1,9 bis 2,0 mg/ml; Konservierung mit Phenoxyethanol 0,3 %: Roggen"Plazenta"-Extrakt I (Tabelle 3) [im Falle der Verwendung des Extraktes in Oralkapsein als Nahrungsergänzungsmittel erfolgt die Konservierung mit Nipagin (p-Hydroxy-benzoresäure-methylester)]

Die aus den beiden Zellkulturen geernteten Zellmassen werden vereinigt und in Diethylether (4 Teile Ether, 1 Teil Wasser) 14 Tage bei -20 °C aufbewahrt. In einer explosionssicheren Trommelzentrifuge wird in einen Rückstand und eine wässrige Phase zerlegt. Letztere wird abgesaugt und geteilt: Lösung I konserviert mit 0,3 % Phenoxyethanol.

Die eine Hälfte der Lösung I wird unter Rühren bei Raumtemperatur mit 0,1 bis 0,5 N NaOH behandelt: Lösung II; die andere Hälfte wird entsprechend behandelt mit 0,1 bis 0,5 N HCl : Lösung III.

Beide Lösungen (II+III) werden miteinander vereinigt und der pH-Wert wird auf 6,7 eingestellt. Beim Filtrieren erhält man eine Lösung IV und den Rückstand II. Der Rückstand II wird mit einer Lösung aus Butylenglycol : Ethanol : Wasser (5:1: 1) bei Raumtemperatur 24 h lang extrahiert. Das Lösungsmittelgemisch wird in einem Vakuum von 0,1 Torr abgezogen, der flüssige Rückstand wird zur Lösung IV zugegeben und der pH-Wert wird auf 6,7 eingestellt. Nach der Filtration werden aus dem Filtrat mit einem Gemisch aus Säuren des Citronensäurecyclus (Citronensäure : Apfelsäure : Bernsteinsäure (2 : 0,3 : 1) Restproteine ausgefällt. Das Filtrat der Lösung V wird mit Lösung IV vereinigt und mit Bernsteinsäure wird der pH-Wert auf 6,7 eingestellt (vgl. Übersicht zu 1.1). Das Lösungsgemisch IV/V wird analysiert und auf die gewünschten Standardwerte (Nachregulierung zur Einstellung auf die gewünschten Standardwerte in bezug auf Trockenrückstand, Stickstoff-Gehalt und pH-Wert auf an sich bekannte Weise) durch Verdünnen mit destilliertem Wasser unter Zusatz des Konservierungsmittels Phenoxyethanol eingestellt etc. (vgl. Tabelle 3, Extrakt I).

1.2 Ein anderer Weg der Extraktion der Zellmassen aus Secale cereale verläuft wie folgt:

## Tafel. II: Übersicht zu 1.2

**Zellmasse aus Fruchtschalen-Plazenta + Zellmasse aus Aleuronschicht**

**(Carpel von Secale cereale)**

↓

**Vereinigen und Behandeln mit Butylenglycol/Ethanol/Wasser**

↓

**Lösungsmittel zum Teil im Vakuum 0,1 Torr abziehen**

↓

**flüssiger Rückstand III**

NaOH        HCl

**Extraktlösung VI        Extraktlösung VII**

**Vereinigen, pH auf 6,7 mit Bernsteinsäure**

↓

**Filtrat: Lösung VIII**

Nach Analyse (Standardisierung), z.B. Regulation des Trockenrückstandes auf die Konzentration von ca. 1,6 bis 1,8 %. N auf 1,9 bis 2,0 mg/ml und Konservierung mit Phenoxyethanol bis auf 0,3 % wird sterilfiltriert: 0,2 $\mu$m Millpore®: Roggen"Plazenta"-Extrakt II (vgl, Tabelle 3).

Tabelle 3
analytische Daten der Roggen-"Plazenta"-Extrakte I und II

|  | Extrakt I | Extrakt II |
|---|---|---|
| pH-Wert | 6,77 | 6,79 |
| Trockensubstanz (%) | 1,66 | 1,65 |
| N (mg/ml) | 1,92 | 1,94 |

(fortgesetzt)

| Identifikation | | |
| --- | --- | --- |
| WARBURG-Test | Faktor > 1,8 | Faktor > 1,8 |
| Aminosäuren | positiv (Ninhydrin) | positiv (Ninhydrin) |
| Peptide | positiv (Biuret) | positiv (Biuret) |
| Nucleinsäure-Komponenten | positiv (TLC) | positiv (TLC) |
| Melanoma B 16-Test | positiv | positiv |
| Reinhelt | | |
| Schwermetalle | < 20 ppm | < 20 ppm |
| As | < 2 ppm | < 2 ppm |
| Sterilität (Phenoxyethanol) | keimfrei (0,3 %) | keimfrei (0,3 %) |

Die toxikologischen und pharmakologischen Daten der Roggen-Plazenta-Extrakte der Tabelle 3 sind folgende:

| | |
| --- | --- |
| akute Toxizität an Mäusen (140 Tiere): | DL 50 i.v. > 25 ml/kg |
| | i.p. > 50 mg/kg |
| | p.o. > 25 g/kg |
| akute Toxizität an Ratten (90 Tiere): | DL 50 i.v. > 25 ml/kg |
| | i.p. > 20 mg/kg |

Studie der chronischen Toxizität an Albinoratten (40 Tiere): keine Toxizität Toxizität bei Hunden (10 Beagle-Hunde, 26 Wochen alt): keine Toxizität Untersuchung der Reproduktion, Fertilität und Teratogenität bei Ratten (80 Tiere): keine Beeinträchtigung Toxizitätsstudlen, peri- und postnatal an Ratten (30 Tiere, 21 Tage alt) Schwangerschaft (Alter der Tiere ca. 100 Tage): keine negativen Befunde Untersuchung der teratogenen Wirkung bei Kaninchen (20 Tiere, Körpergewicht ca. 2,4 kg): keine negativen Befunde

1.2 <u>Testversuche mit Roggen-Plazenta in Extrakten zur Demonstation ihrer Wirksamkeit</u>
1.2.1 Wundhellungsversuche, Hautspannungsmessungen
1.2.2 Atmungssteigerung (WARBURG)
1.2.3 Anwendung der PADBERG-Methode
1.2.4. Wirkung auf menschliche Fibroblasten und Keratinozyten (MTT-Test).

ad 1.2.1 <u>Wundheilungsversuche, Hautspannungsmessungen</u>

Unter anderem waren zur Demonstration der Wirksamkeit des erfindungsgemäßen Extraktes Wundheilungsversuche bei Ratten gemäß DE-PS 37 11 054 sowie anschließend Hautspannungsmessungen durchgeführt worden (vgl. auch EP-A-0 552 516, Tabelle II). Diese Ergebnisse sind in der folgenden Tabelle 4 aufgeführt:

Tabelle 4: Ergebnis der Hautspannunasmessungen (Spalten II und III)

| Spalte I | Spalte II | Spalte III | Spalte IV |
| --- | --- | --- | --- |
| Zeit nach dem Setzen der Wunde | Versuchsgruppe | Kontrollgruppe | Differenz $\Delta$ |

a) Roggen-"Plazenta"-Extrakt I (Tabelle 3)

| | | | |
| --- | --- | --- | --- |
| 6 Tage | 250 | 140 | 110 |
| 12 Tage | 510 | 410 | 100 |
| 18 Tage | 1500 | 1210 | 290 |

Die in der Tabelle 4 aufgeführten Ergebnisse der Hautspannungsmessungen dokumentieren die überlegene Wirksamkeit des erfindungsgemäßen Präparats gegenüber der Kontrollgruppe.

Ergebnis der Warburg-Versuche:

Atmungssteigerungsfaktoren der Roggen-"Plazenta"-Extrakte I und II

|  |  |  |  |
|---|---|---|---|
| Extrakt | I | : | 1,95 |
| Extrakt | II | : | 2,05 |

ad 1.2.2: Atmungssteigerungsversuche

Die Atmungsstelgerungs-Faktoren sind nach folgender Arbeitsvorschrift über "Wirksamkeitsprüfung von Testlösungen auf Atmungssteigerung im Rattenleberhomogenat (Warburg-Methode)" gewonnen worden.

Methode und Arbeitsplan

A. Lösungen

1. Sörensen-Puffer, pH 7,4 aus 80,4 ml einer Lösung von 11,87 g $Na_2HPO_4.2H_2O/l$ und 19,6 ml einer Lösung von 9,08 g $KH_2PO_4/l$.
Nach Zusammengeben der genannten Lösungsmengen wird der pH-Wert überprüft und, falls erforderlich, durch Zusatz der einen oder anderen Lösung korrigiert.

2. Rattenleberhomogenat: Einer 24 h-Hungerratte wird nach Dekapitation und Ausbluten die Leber entnommen und sofort auf Eis gekühlt. Nach dem Waschen und Abtrocknen mit Zellstoff werden 6 g zerkleinerte Leber im Homogenisator nach Potter-Elvehjem mit leichtgängigem Teflonpistill auf 30 ml mit gekühltem Sörensenpuffer aufgefüllt und unter Eiswasserkühlung für 2 bis 3 min homogenisiert. Das fertige Homogenat läßt man für ca. 3 min unter Eiskühlung stehen, um die beim Homogenisieren eingequirite Luft entweichen zu lassen.

3. KOH 6N

4. Testlösungen dieser Patentanmeldung, z.B. Extrakt I aus den Tabellen 3 und 4

5. Aqua dest.

B. Versuchsdurchführung

Kegelförmige Warburg-Gefäße mit einem Zylindereinsatz (ZE) und einem Seitenarm (SA) und einem Volumen von ca. 13 ml werden wie folgt gefüllt:
Füllpian, die Angaben erfolgen in ml.

| Gläser Nr. |  | 1/2 | 3/4 | 5/6 | 7/8 |
|---|---|---|---|---|---|
| Lösung | Füllort |  |  |  |  |
| 1 | HR* | 3,0 | 1,5 | 3,0 | 1,5 |
| 5 | SA | 0,25 | 0,25 | - | - |
| 4 | SA | - | - | 0,25 | 0,25 |
| 3 | ZE | 0,2 | 0,2 | 0,2 | 0,2 |
| 2 | HR | - | 1,5 | - | 1,5 |
| * HR = Hauptraum |  |  |  |  |  |

Die Lösungen werden in der angegebenen Reihenfolge einpipettiert. Zur Erhöhung der KOH-Oberfläche werden in die Zylindereinsätze gefaltete Filterstreifen eingesetzt. Sofort nach Beendigung der Füllung werden die Gefäße mit den passenden Schlauchventilstopfen am Seitenarmansatz dicht verschlossen, die Gefäße an den Manometern befestigt und mit Klammern oder Paketgummis gesichert. Die Manometer werden bei geöffneten Hähnen In die auf 37 °C temperierte Apparatur eingehängt und 60 min geschüttelt, um die Reaktlonslösung zu temperieren und die anfänglich sehr schnelle Atmung abklingen zu lassen. Danach werden die rechten Manometerschenkel mit Hillfe des Quetschhahns auf die Marke "15" eingestellt, die Differenz der linken Schnekel zu "15" notiert, die Manometerhähne geschlossen und die Lösungen aus den Seitenarmen durch Kippen in die Haupträume überführt (0-Zeit). Nach je 20 min wird die Schüttelvorrichtung abgestellt, die rechten Manometerschenkel auf "15" eingestellt (V = konst.) und die Differenz der linken Schenkel zu "15" notiert.

Der Versuch läuft bis zu einer Messdauer von 100 min.

Sollte in einigen Gläsern der Sauerstoffverbrauch so groß sein, dass die Manometerlänge nicht ausreicht, wird rechtzeitig ein Druckausgleich geschaffen, indem man sofort nach der Ablesung den Manometerhahn öffnet, den rechten Schenkel wieder auf "15" einstellt, die Differenz des linken Schenkels zu "15" notiert und den Manometerhahn sofort wieder schließt. Nach der letzten Ablesung werden die Manometerhähne geöffnet und die Volumina der Gefäße und der dazugehörigen Manometer notiert.

Auswertung

Zunächst werden die Gefäße 3/4 und 7/8 mit den entsprechenden Thermobarometern (TB: Gefäß 1/2 bzw. 5/6) abgeglichen, um Luftdruck- und Temperaturschwankungen während des Versuchs auszugleichen. Danach werden die Gefäßkonstnaten für $O_2$ der Gläser 3/4 und 7/8 nach folgender Gleichung berechnet:

$$K = \frac{V_g \cdot (273/T) + V_f \cdot \alpha}{P_0}$$

$V_g$ = Volumen der Gasphase (Volumen des Gefäßes und des Manometers bis zur Marke "15" abzüglich $V_f$).

$V_f$ = Volumen der im Gefäß enthaltenen Gesamtflüssigkelt. T = Versuchstemperatur in °K, $\alpha$ = Bunsenscher Absorptionskoeffizient ($O_2$ 37 °C = 0,0239).

$P_0$ = 1 atm, gemessen in mm Brodie'sche Lösung (Monometerfüllung) bei 0,1 MPa (760 mm Hg) = 10 0000 Brodie'sche Lösung.

Der verbrauchte Sauerstoff kann nun direkt nach der Gleichung

$$X (\mu l\ O_2) = h \cdot k$$

berechnet werden, da die durch die Atmung entstandene Kohlensäure durch die KOH vollständig absorbiert wurde (h = manometrische Differenz). Dividiert man die verbrauchten $\mu l\ O_2$ der Gläser 7/8 durch die der Gläser 3/4, so erhält man einen Faktor, der das Maß der Atmungssteigerung darstellt.

ad 1.2.3: Anwendung der Padberg-Methode

Die kosmetische Wirksamkeit des zu prüfenden Extrakts wurde nach der Padberg-Methode ermittelt. Getestet wurden eine W/Ö-Creme mit 2 % Extrakt und zur Kontrolle eine Creme ohne Extrakt-Zusatz. Der Padberg-Test wurde wie folgt durchgeführt:

- Markieren der Teststelle auf der Haut
- Vorbehandlung der Hautstelle mit einer 1 %igen Lösung eines nlchtlonischen Tensids zur Beeinträchtigung der Haut ("subjektiv raue Haut")
- Waschen der Teststelle mit 37 °C warmem Wasser
- Abwischen der Teststelle
- Akklimatisieren des Subjekts bei 20°C und einer relativen Feuchte von 60 % für eine Zeitdauer von 45 min
- Auftragen von 20 ml einer Flecklösung, die 20 % 0,5 %iges Methylenblau und 80 % eines 1 %igen nichtionischen Tensids enthält

- 30 Sekunden langes Einwirken der Flecklösung auf die Teststeile
- Trocknen des Farbstoffs, 1 min lang
- Entfernen von überschüssigem Farbstoff mit einer 0,2 %Igen Lösung des verwendeten nichtionischen Tensids
- 2-maliges, jeweils 60 Sekunden langes Eluieren mit 2 ml-Portionen einer Natriumlauratlösung (2,3 % Natriumlaurat, 48,7 % reines Wasser, 49 % Isopropylalkohol)
- Bestimmung der Absorption des Eluats bei 660 nm mit einem Spektrometer

Die Testbehandlung wurde durchgeführt, indem über einen Zeitraum von 20 Tagen zweimal täglich eine Probe aufgetragen wurde. Dabei wurde sichergestellt, dass die Testpersonen sowohl 3 Tage vor dem Test als auch während der Testperiode kein anderes Kosmetikum verwendet hatten. Am 21. Tag nach Beginn der Behandlung wurden die oben aufgeführten Verfahrensstufen einschließlich der Elution 4 Stunden vor der abschließenden Bewertung durchgeführt.

In der folgenden Tabelle 5 ist das jeweilige Verhältnis der Absorption von Methylenblau vor und nach dem Auftragen bei jeder Person für die W/Ö-Cremes in % aufgeführt, wobei die Extrakt-haltige Creme mit der Kontroll-Creme verglichen wurde.

Die absorbierte Menge an Methylenblau verläuft proportional zur Rauheit der Haut und die in der Tabelle aufgeführten Prozentgehalte wurden gemäß der folgenden Gleichung ermittelt:

$$\text{Hautraulgkeit \%} = \frac{\text{Absorption nach Auftragung}}{\text{Absorption von unbehandelter Haut}} \times 100,$$

wobei davon ausgegangen wird, dass eine mit einer Creme behandelte Haut nur eine kleine Menge Methylenblau absorbiert. Die Mittelwerte für 21 Versuchspersonen ergaben, dass der Roggen-"Plazenta"-Extrakt (Spalte I) gute Rauigkeitswerte gegenüber der Kontroll-Creme (Spalte II) zeigt.

Tabelle 5

| Alter der Versuchsperson | I Extrakt-haltige Creme | II (Kontrolle) |
|---|---|---|
| 41 | 60,1 | 95,0 |
| 45 | 55,0 | 80,0 |
| 62 | 40,1 | 91,2 |
| 45 | 58,1 | 83,0 |
| 66 | 40,8 | 92,4 |
| 40 | 55,2 | 78,9 |
| 46 | 53,9 | 96,0 |
| 62 | 61,8 | 88,5 |
| 63 | 42,7 | 90,1 |
| 69 | 40,1 | 73,9 |
| 51 | 54,2 | 90,1 |
| 60 | 58,5 | 31,0 |
| 70 | 54,0 | 78,0 |
| 67 | 60,0 | 89,6 |
| 42 | 61,8 | 80,8 |
| 47 | 39,8 | 72,9 |
| 60 | 46,9 | 76,6 |
| 65 | 62,9 | 76,5 |
| 50 | 64,0 | 94,6 |
| 41 | 60,7 | 91,4 |
| 46 | 52,4 | 95,0 |

Bestimmung des Whitening-Effekts von Roggen-"Plazenta"-Extrakt (Extrakt in einer Handcreme-Emulsion, die Zusammensetzung der Handcreme ist nachstehend angegeben).

Ergebnis: Nach Zerstörung der Emulsion der Roggen-"Plazenta"-Extrakthaltigen Handcreme durch Wasserzusatz und durch Erhitzen auf 80°C wurde die wässrige Phase abgetrennt, im Vakuum eingeengt und der Zentrifugenrückstand wurde gefriergetrocknet.

Eine definierte Menge des dabei erhaltenen Lyophilisats wurde in entlonisiertem Wasser gelöst und wie nachstehend angegeben weiter behandelt.

B16-Melanoma-Zellen von Mäusen wurden überimpft und 6 Tage lang kultiviert mit der Testprobe bzw. Kontrolle bei 37 °C, 5 % $CO_2$ + 95 % Luft, wobei das Kulturmedium am dritten Tag gewechselt wurde.

Die Beurteilung der Whitening-Effekte wurde vorgenommen nach Abtrennung der Zellen unter Verwendung von 6 ml EDTA-Lösung und Auszählung der Zellen.

Tabelle 6

| Testproben Konzentration in mg/ml | Anzahl der Zellen | Relativwert | Whitening-Effekt |
|---|---|---|---|
| 2,6 | $78 \times 10$ | 0,95 | +++ |
| Kontrolle | $90 \times 10$ | 1,00 | - |

Zusammensetzung der Handcreme

5 % acetylierte Lanoninalkohole, 1 % Lanolin-Derivat (lösllch), 5 % Multisterol-Extrakt, 5 % Vaseline, 0,75 % Pvlyvinylalkohol-Gel, 10 %iges NaOH, 3,75 % ethoxylierte Fettamine, 0,3 % Roggen-"Plazenta"-Extrakt, 0,2 % Parfümöl, Wasser bidest. ad 100 %.

Beurtellungskriterien

a) Skala zur Bewertung des Whitening-Effekts (Melanin-Hemmung)

| | |
|---|---|
| wie die Blindkontrolle | - |
| leichter Whitening-Effekt | + |
| ausgeprägter Whitening-Effekt | ++ |
| starker Whitening-Effekt | +++ |

Reihenfolge der Testverfahrensmaßnahmen:

1. Herstellung und Sterilisierung eines Test-Kulturmediums
2. Herstellung einer Zellenflotationslösung
3. Einstellung des pH-Wertes des Kulturmediums
4. Ausstreichen des Kulturmediums auf einer Petrischale
5. Einimpfen von $B_{16}$-Melanoma-Zellen von Mäusen
6. Kultivieren in einem Thermostaten (37 °C, 5 % $CO_2$, 95 % Luft)
7. Auswechseln des Kulturmediums am 3. Tag
8. Kultivieren in dem Thermostaten (37 °C, 5 % $CO_2$, 95 % Luft)

Tabelle 7: $B_{16}$ Melanoma-Zellen-Test-Daten

| Probe | Menge | Whitening-Effekt | Anzahl der Zellen |
|---|---|---|---|
| Roggen-"Plazenta"-Extrakt | 0,250 | +++ | $79 \cdot 10^4$ |
| " | 0,200 | ++ | $83 \cdot 10^4$ |
| Kontrolle | 0 | $\pm$ | $88 \cdot 10^4$ |

ad 1.2.4. <u>Zellaktivierungseffekt auf menschliche Fibroblasten (MTT-Test)</u>

Normale menschliche Hautfibroblasten wurden bei 37 °C in einer Atmosphäre aus 5 % $CO_2$ und 95 % Luft in Dulbecco's modifiziertem Eagle's Medium (DMEM) unter Zusatz von 1 % FBS (fetalem Rinderserum) gezüchtet, wobei für den Test die fünfte und siebte Passage verwendet wurden.

Zur Kontrolle wurde die Züchtung in DMEM-Medium + 1 % FBS ohne CR-1 durchgeführt.

<u>MTT-Assay</u>

Menschliche Fibroblasten wurden im MTT-Assay eingesetzt, um die Zellstoffwechselaktivierung durch Roggen-Plazenta-Extrakt zu bewerten:

Die Jeweiligen Zellen wurden in ihrem oben definierten Medium, das varlierende Mengen an Roggen-Plazenta-Extrakt (CR-1) enthielt, bzw. keinen Roggen-Plazenta-Extrakt (CR-1) enthielt (Kontrolle), 48 h lang gezüchtet. Nach der Entfernung des Mediums wurde das in den Zellen gebildete blaue Formazan in 200 $\mu$l 2-Propanol solubilisiert. Die Bestimmung des Formazans erfolgte durch Messung der Extinktion bei 550 m unter Verwendung eines Mikroplate-Reader. Die Ergebnisse sind in dem nachfolgenden Diagramm dargestellt.

## Diagramm: Stoffwechselaktivierung durch Roggen-Plazenta-Extrakt (CR-1) bei Human-Fibroblasten (MTT)

**Messwerte zum obigen Diagramm**

| CR-1-Konzen-tration(%) | Aktivierungsindex (%) Mittel-wert | ± | Standard-abweichung (SD) |
|---|---|---|---|
| DMEM+1 % FBS+0 | 100.0 | ± | 2.2 |
| DMEM+1% FBS+ 0.004 | 105.7 | ± | 3.6 |
| " 0.008 | 104.7 | ± | 2.7 |
| " 0.016 | 105.2 | ± | 2.5 |
| " 0.031 | 105.3 | ± | 2.5 |
| " 0.063 | 105.1 | ± | 3.1 |
| " 0.125 | 104.7 | ± | 2.3 |

<u>Beispiel 2</u>

<u>Weiterverarbeitung des Rückstandes I aus Beispiel 1 (Übersicht zu 1.1)</u>

Das mechanisch zerschnittene und zerkleinerte Pflanzenorganmaterial des Rückstandes I aus Beispiel 1.1 wird in Chargen zu 5 kg in einem Citrat-Succinat-Puffer von pH 6,5 suspendiert und in einem Kilowatt-Ultraschall-Generator, der starke Oszillationen liefert, bearbeitet. Der gewonnene Zentrifugenrückstand enthält das gewünschte Zellwandmaterial, das als Basis für die folgenden enzymatischen und chemischen Hydrolysen-Prozesse dient.

Nach 5-tägiger Behandlung mit 10 % Cellulase bei 40 °C wird zentrifugiert. Der Rückstand I wird bei 4 °C für die weitere Behandlung aufbewahrt. Die überstehende Lösung I wird mit Schwefelsäure auf eine Konzentration von 2 N gebracht und zur partiellen Hydrolyse 5 Tage lang bei 15 at autoklaviert (4 bis 9 °C). Bei der Trennung erhält man den Rückstand II und die Lösung II, die auf pH 5,9 gebracht wird. Die Rückstände I und II werden partiell im Autoklaven in 3 N HCl hydrolysiert und dann mit Hydrolysat II (aus I) vereinigt und dialysiert. Nach dem Einengen des Dialysats im Vakuum bei 20 °C auf ca. 3 % Trockenrückstand erfolgt Einstellung auf pH 6,7 und Sterilfiltration (0,2 $\mu$m).

Der resultierende Roggen-"Plazenta"-Extrakt III weist folgende Eigenschaften auf:

pH 6,7; Trockenrückstand 2,9 %; N 3,1 mg/ml; Aminosäuren: TLC- und Ninhydrin-positiv; Stoffwechselaktivität WARBURG-Faktor: > 1,8; Sterilität: keimfrei (0,3 % Phenoxyethanol); Protein-frei.

Beispiel 3

Herstellung eines öllöslichen Roggen-"Plazenta"-Extrakts

Extraktion A

Das in Beispiel 2 zerkleinerte und mit Ultraschall aufgeschlossene Pflanzenorganmaterial des Rückstands I (aus Beispiel 1) wird unter $N_2$ bei Raumtemperatur 30 Tage lang mit der 10-fachen Menge Olivenöl/Isopropylmyristat/Sonnenblumenöl (4,5 : 1 : 0,02) ausgezogen und durch ein geeignetes Filter von ungelösten Bestandteilen befreit: Extrakt I.

Extraktion B

Getrocknete Zellmassen aus Fruchtschalen-Plazenta und Aleuronschicht werden unter $N_2$ wie unter A beschrieben 30 Tage lang mit Olivenöl/Isopropylmyristat/Sonnenblumenöl ausgezogen; nach der Filtration erhält man den Extrakt II.

Die unter $N_2$ gelagerten Extrakte I und II werden vereinigt und analysiert; schließlich erhält man den Roggen-"Plazenta"-Extrakt III.

Der öllösliche Roggen-"Plazenta"-Extrakt lässt sich ausgezeichnet in Fettcremes und Ölen (Emulsionen) vom W/O-Typ einarbeiten, sodass die Plazenta-Inhaltsstoffe in der Ausgangsphase der Emulsion voll zur Geltung kommen. Es handelt sich dabei um ein bräunliches Öl von typischem schwachem Geruch, der jedoch die Parfümierung der Kosmetika bei einem Zusatz bis zu 2 % nicht beeinflusst.

Der öllösliche Extrakt ist standardisiert. Die für die Beurteilung und Betriebskontrolle wichtigen Kennzahlen sind die Verseifungszahl und der Gehalt an fettlöslichen Vitaminen, wie z.B. Vitamine A, $D_2$, E, $K_3$, Provitamine u.a. Der Extrakt ist frei von Hormonen (siehe Qualitätsstandard). Der Extrakt ist so stabilisiert (Antioxidantien), dass eine Haltbarkeit von einem Jahr garantiert ist.

Eigenschaften (Übersicht)

| | |
|---|---|
| Verseifungszahl | > 120 |
| Jodzahl | > 20 |
| Vitamine, z.B. | A, $D_2$, E, $K_3$ |
| ferner Provitamine | Carotinoide |
| | Phytosterine |
| ungesättigte Fettsäuren | Linolsäure |
| | Linolensäure |
| | Arachidonsäure |
| Hormone | $C^{17}$-Ketasteroide |
| | nicht nachweisbar |
| Sterilität | keimfrei |
| Toxizität | |
| $DL_{60}$ p.o. | 50 ml/kg Maus |

Aufgrund einer akuten Toxizitätsprüfung, und zwar durch einmalige orale Applikation, erweist sich der Extrakt als ungiftig. Die $DL_{50}$ beträgt > 50 ml/kg Maus.

Jodzahl des öllöslichen Extrakts (nach Wijs)

Aussage: die Jodzahl gibt an, wie viel g Halogen, berechnet als Jod, von 100 g Substanz gebunden werden.

Reagentien: Tetrachlorkohlenstoff, Wijs-Lösung (Jodmonochlorid-Lösung), Kaliumjodld-Lösung 25 %ig, Stärke-Lösung, 0,1 N Natriumthiosulfat-Lösung.

Analyse: 0,25 bis 0,3 g (genau gewogen) Extrakt werden in einem 250 ml Jodzahikolben in 10 ml Tetrachlorethan gelöst, mit 25 ml Wljs-Lösung versetzt und 30 min im Dunkeln verschlossen stehen gelassen. Danach fügt man 10 ml 25 %ige KJ-Lösung und 100 ml $H_2O$ hinzu und titriert sofort unter kräftigem Schütteln mit 0,1 N $Na_2S_2O_3$ bis zur hellbraunen Färbung; nach Zusatz von ca. 5 ml Stärke wird die Titration bis zur völligen Entfärbung fortgesetzt. Ein Blindversuch wird in gleicher Weise behandelt.

$$\text{Berechnung:} \quad \frac{(\text{ml } 0,1 \text{ N } Na_2S_2O_3 \text{ Blind} - \text{ml } 0,1 \text{ N } Na_2S_2O_3 \text{ Probe} \times 1,27)}{\text{Einwage}} = \quad \text{Ergebnis: im Beispiel der vorstehenden Selte}$$

Nachtcreme vom Typ W/O

| | |
|---|---|
| Isopropylstearat | 16,0 % |
| Eutanol G | 15,6 |
| Paraffinöl perl. | 5,0 % |
| Lunacera M | 7,0% |
| Aluminiumstearat DF 30 | 2,0 % |
| Lameform GE 2 | 5,0 % |
| Lunacera alba | 7,0% |
| Borax | 0,5% |
| Glycerin | 3,0% |
| Dowicil 200 | 0,2% |
| Plazenta-Extrakt, öllöslich | 3,0 % |
| Parfümöl | 0,5% |
| Oxynex 2004 | 0,1 % |
| Wasser | ad 100,0 % |

Herstellungsvorschrift

Aluminiumstearat und Paraffinöl solange erhitzen, bis das Stearat vollständig gelöst ist. Anschließend erfolgt die Zugabe von Lunacera M, Lameform GE 2, Isopropylstearat, Eutanol G, Lunacera alba und Oxynex. Die Fettphase wird auf ca. 80 °C erhitzt.

Die Wasserphase, bestehend aus Glycerin, Borax und Dowicil, ebenfalls auf 80 °C erhitzen und unter anfänglich schnellem Rühren langsam der Fettphase zugeben.

Bei ca. 30 °C erfolgt die Zugabe von Plazenta-Extrakt und Parfümöl. Die fertige Emulsion wird bei Stufe 2 homogenisiert.

Beispiel 4

Herstellung eines Reis-Plazenta-Extrakts (Reis: Oryza sativa)

Die von Dr. A. Brown 1990 in der Abteilung Biochemie des Chemischen Zentralinstituts der Brasilianischen Bundesunlversität S. Maria (UFSM) hergestellte, später tiefgefroren gelagerte Rels-Plazenta-Zellkultur [aus den geschnittenen Fruchtblättern des Fruchtknotens der Samenanlage] diente als Ausgangszelikultur. Die aufgetaute Zellkultur wurden in üblicher Weise in eine modifizierte Nährlösung gebracht und es wurde dann nach und nach in verschiedenen Systemen eine ausreichende Zellmasse gezüchtet. Die Zellmasse wurde einige Zeit mit einem Gemisch aus Butylenglycol/Ethanol/$H_2O$ behandelt, das man dann im Vakuum von ca. 0,1 Torr abzieht. Der pH-Wert des flüssigen Destillationsrückstandes wird mit Bernsteinsäure auf pH 6,8 eingestellt und nach dem Abfiltrieren von restlichen Proteinen analysiert, gegebenenfalls auch dialysiert. Nach Einstellung des Trockenrückstandes durch Verdünnen mit destilliertem Wasser oder Einengen im Vakuum von 0,1 Torr werden die N-Konzentration und die Konzentration des Konservierungsmittels Nipagin (4-Hydroxy-benzoesäure-methylester) auf 0,3 % und der pH-Wert auf 6,8 eingestellt. Dann wenn eine Sterilfiltration durch Millipore 0,2 $\mu$m durchgeführt. Die dabei erhaltenen analytischen Daten sind wie folgt:

| | |
|---|---|
| pH-Wert | 6,82 |
| Trockensubstanz | 1,8 % |
| N-Gehalt | > 1,8 mg/ml |
| Warburg-Faktor | > 1,8 |
| Aminosäuren | positiv (Ninhydrin) |
| Peptide | positiv (Biuret) |
| Protein | negativ |
| Schwermetalle | < 20 ppm |
| AS | < 2 ppm |
| Sterilität | keimfrei, Prionen-frei |
| Toxikologie: | keine negativen Befunde (analog zu Beispiel 1) |
| Whitening-Effekt | +++ |

Testversuche

1. Der Reis-"Plazenta"-Extrakt, eingearbeitet in einer Menge von 1 bis 3 % in eine W/Öl-Emulsion reguliert und verbessert das Feuchthaltevermögen (Humidity-maintaining-capacity) der menschlichen Haut: vgl. die weiter unten folgende Tabelle 9: Hautfeuchtigkeltsmessungen.

2. Nachweis des "Whitening-Effekts" nach Anwendung einer Reis-"Plazenta"-Extrakt-Formulierung nach Vorschrift analog Beispiel 1.

3. Einfluss des Reis-Plazenta-Extrakts auf das Wachstum und die Metamorphose von Kaulguappen (Xenopus laevis DAUDIN)

Das Wachstum von Kaulquappen wurde vom Tag 1 (vom Froschpaar abgelegter Laich) bis zum Tag 66 beobachtet und ab Tag 3 gemessen, nachdem die frei schwimmenden Kaulquappen erhalten worden waren. Wassertemperatur 24 °C. Am Tag 7 wurden die Kaulquappen in zwei Gruppen aufgeteilt, um einen Test mit dem Extrakt und einer Kontrolle durchzuführen. Jede Versuchsgruppe umfaßte ca. 150 Kaulquappen.

Die Wassermenge wurde gesteigert auf 2,5 L bis zum Tag 21, auf 5 L bis zum Tag 35 und auf 7,5 L pro Gruppe ab Tag 36 bis 67. Belüftung mittels handelsüblicher poröser Aquariensteine. Wasserwechsel der Becken alle 7 Tage. Futter ab Tag 7 Brennesselpulver, vorher Liquifry.

Zugabe des Extrakts pro Woche in 3 Portionen, Menge 0,2 %. Längenmessungen hier nicht notiert.

Beginn der Metamorphose beim Extrakt am Tag 50, bei den Kontrollen erst am Tag 56: vgl. Tabelle 8.

Die Frösche von Xenopus laevis sind Carnivoren und erhalten frische Leber als Futter, die Kaulquappen sind Pflanzenfresser (Brennessel).

Tabelle 8

| Metamorphosen und Frösche, d 50-66 | | | | |
|---|---|---|---|---|
| Versuchsgruppe | 1 | 2 | 3 | 4 |
| d50 | | | 1+0 | 1+0 |

(fortgesetzt)

| Metamorphosen und Frösche, d 50-66 | | | | |
|---|---|---|---|---|
| Versuchsgruppe | 1 | 2 | 3 | 4 |
| d53 | | | 1 + 1 | 1 + 0 |
| d56 | 0+2 | | 1+1 | 1+2 |
| d58 | 0+3 | 0+1 | 1 +1 | 1 +2 |
| d60 | 0+3 | 0+1 | 1 +2 | 1 +2 |
| d63 | 0+4 | 1 +1 | 2+3 | 2+4 |
| d64 | 1 +3 | 1 + 1 | 2+5 | 2+5 |
| d66 | 2+2 | 1 +0 | 2+6 | 3+5 |

Erste Zahl:     Anzahl der Frösche

Zweite Zahl:    Anzahl der Metamorphosen

                     (Die Metamorphose ist beendet, wenn der Schwanz vollständig oder fast vollständig resorbiert worden ist)

1, 2:           Kontrollen

3, 4:           Reis-"Plazenta"-Extrakt

Beispiel 5

Gewinnung eines Triticale-"Plazenta"-Extrakts

Verwendung einer Zellkultur aus den Fruchtblättern der Triticale-Samenanlage, gewonnen 1992 in der Abteilung Biochemie des Chemischen Zentralinstituts der UFSM S. Maria R.S., Brasilien und tiefgefroren gelagert ebenda. Auftauen der Zellkultur und Züchten einer ausreichenden Menge Zellmasse in Suspensionskultur. Weiterverarbeitung gemäß Übersicht in der folgenden Tafel 3.

Tafel 3: Gewinnung eines Triticale -"Plazenta"-Extrakts via Zellkultur

Triticale vulgare-Plazenta-Zellkultur (20 ml)

↓

Vermehrung nach der Rotary-Shaker- Technik (500 ml)

↓

in Suspensionsflaschen (5 - 20 L)

↓

in 50 - 1000 L-Fermentern oder

In 2000 L-Rohrschlangen-Kultivatoren

↓

Ernte von Plazenta- Triticale-Zellmaterial in Suspension bzw. Pulverform

Zellwand-Aufbrechen

suspendiertes Zellmaterial im Kilowatt-Ultraschall-Generator zerbrochen

↓

Zentrifugieren des aufgeschlossenen Materials und Waschen mit Wasser

↓

Splitting und Deproteinierung

↓

durch chemische und enzymatische Partialhydrolyse, pH-Einstellung auf 6,7

Filtration und Einengen im Vakuum auf die gewünschte Extrakt-Konzentration

↓

Konservierung mit 0,3 % Phenoxyethanol

↓

Einstellung der Kennzahlen

(fortgesetzt)

↓

Sterilfiltration 0,2 μm.

Analysen und Testversuche nach Prüfung auf toxikologische Eigenschaften:

| Analysendaten: | pH 6,7; N > 1,5 mg/ml |
|---|---|
| Trocken rückstand | 1,9 % |
| Warburg-Faktor | > 1,8 |
| Aminosäuren, Peptide | positiv |
| Proteine | negativ |
| Sterilität | keimfrei |

Hautfeuchtigkeitsmessungen nach einmaliger und nach 14-tägiger Applikation einer Triticale-"Plazenta"-Extrakt bzw. Reis-"Plazenta"-Extrakt enthaltenden Creme (Corneometer, Figuren 4a und 4b).

Triticale-"Plazenta"-Extrakt bzw. Reis-"Plazenta"-Extrakt wurde zu 5 % in die folgende Rezeptur eingearbeitet:

Phase I Schmelzen auf 70°C: C: 10 % Cetiol V, 10 % Lanette N; 5 % Mlglyol 812, 2 % Adeps lanae; 0,3 % Phenoxyethanol:

Phase II auf ca. 70°C erwärmen, enthaltend: 0,3 % Phenoxyethanol; 5 % Karion fl., Karion F; 62,3 % Wasser;

Phase II in Phase I einrühren, weiterrühren bis die Mischung auf 32°C abgekühlt ist, dann Triticale- bzw. Reis-"Plazenta"-Extrakt (5 %) und Parfümöle einrühren und homogenisieren.

Analog wurde eine Creme mit 5 % Kollagen (1 %ig) herstellen, ebenso eine Kontrollcreme ohne Zusatz eines Additivs.

Die Messungen ergaben für das Feuchtigkeitshaltevermögen:

Tabelle 9

| nach einmaliger Applikation getestet | Hautfeuchtigkeitshaltevermögen in Stunden | |
|---|---|---|
| Basis (Kontrolloreme) | 4,6 | 4,6 |
| Basis + 5 % Triticale-"Plazenta"-Extrakt | 6,5 | |
| Basis + 5 % Rels-"Plazenta"-Extrakt | | 7,2 |
| Basis + 5 % Kollagen | 6,4 | 6,4 |

| nach 14-tägiger Applikation: getestet | Verbesserung der Hautfeuchtigkelt in % | |
|---|---|---|
| Basis (Kontrollcreme) | 8,5 | 8,5 |
| Basis + 5 % Triticale-"Plazenta"-Extrakt | 21 | |
| Basis + 5 % Rels-"Plazenta"-Extrakt | | 24,5 |
| Basis + Kollagen 5 % | 19 | 19 |

Einfluss einer Triticale-"Plazenta"-Extrakt enthaltenden Öl/Wasser-Emulsion auf die Haut-Temperatur von Probanden mit trockener Haut.

Der Einfluß der Emulsion auf die Hauttemperatur wurde an 30 Probandinnen im Alter von 40 bis 60 Jahren mit trockener Haut getestet. Die Ergebnisse sind in der nachfolgenden Tabelle 10 angegeben.

Tabelle 10

| Temperatur (°C) vor Applikation | Öl-Wasser-Emulsion | Temperatur (°C) nach Applikation | ΔT |
|---|---|---|---|
| 30,6-33,0 | Triticale-"Plazenta"-Extrakt | 32,7 - 32,9 | 0,2 |
| 30,5 - 34,0 | Kontrollemulsion | 30,5 - 33,6 | 3,1 |

Ohne Triticale-"Plazenta"-Extrakt konnte keine merkliche Stabilisierung (kleinerΔT-Wert) der Hauttemperatur festgestellt werden, daher hoher ΔT-Wert bei Verwendung der Kontrollemulsion.

Nach folgender Vorschrift wurde eine Lanolin enthaltende Handcreme formuliert, in die Triticale-"Plazenta"-Extrakt inkorporiert wurde. Die End-Zusammensetzung der Creme-Formulierung war folgende:

| | |
|---|---|
| lösliches Lanolinderivat | 1,0 Gew.-% |
| acetylierte Lanolinalkohole | 5,0 Gew.-% |
| flüssiger Multisterol-Extrakt | 5,0 Gew.-% |
| Vaseline | 5,0 Gew.-% |
| Polyvinylalkohol-Gel | 0,75 Gew.-% |
| 10 %iges NaOH | 2,25 Gew.-% |
| Triticale-"Plazenta"-Extrakt | 6,0 Gew.-% |
| ethoxylierte Fettamine | 3,75 Gew.-% |
| Parfümöl | 0,3 Gew.-% |
| Wasser, bidest. | ad 100,00 Gew.-% |

Zur Herstellung dieser Handcreme wurden die Fettphasen-Bestandteile und Vaseline auf ca. 75°C erwärmt. Gleichzeitig wurde das PVA-Gel in heißem Wasser von ca. 80°C dispergiert und alimählich gelöst. Zur letzteren Dispersion wurden das ethoxylierte Fettamin (mit ca. 25 EO-Einheiten) und gegebenenfalls noch ein Emulgator gegeben, wonach die vorher angesetzte erwärmte Fettphase darin emulgiert wurde. Nach 5 Minuten Rühren bei der Mischungstemperatur wurde auf 60°C abgekühlt, dann mit 10 %iger Natronlauge versetzt und weiter bis auf unter 40°C gekühlt. Unter langsamem Rühren wurden dann 6 % Triticale-"Plazenta"-Extrakt sowie das Parfüm zugesetzt. Der Ansatz wurde gut homogenisiert. Die Handcreme war stabil konfektioniert und zeigte eine verbesserne Hautkonditionierung, verglichen mit einem Ansatz ohne Triticale-"Plazenta"-Extrakt.

Eine Creme-Grundlage wurde wie folgt compoundiert:

| | |
|---|---|
| Vaseline | 40 Gew.-% |
| Cetanol | 18 Gew.-% |
| Sorbitansesquioleat | 5 Gew.-% |
| Polyoxyethylenlaurylether | 0,5 Gew.-% |
| p-Hydroxyalkylbenzoat | 0,2 Gew.-% |
| Creme-Grundlage mit Triticale-"Plazenta"-Extrakt | 5,0 Gew.-% |
| Wasser | ad 100,00 Gew.-% |

Beispiel 6

Herstellung eines Roggen-Plazenta-Extrakts aus frischen Roggen-Plazenten in Kombination mit einem in Beispiel 1 beschriebenen Roggen-Plazenta-Extrakt auf Zellkulturbasis

Ausgangsmaterial: gut gewaschene Roggen-Fruchtschalen, die Plazenta-Material enthalten, werden erneut gewaschen, zerkleinert (Verfahrensvariante A) und mit Butylenglycol/Ethanol/Wasser behandelt (vgl. die Überschrift zu 1.2 In Beispiel 1). Zur weiteren Extraktion wird verfahren wie ebenda beschrieben.

Die aus 100 kg frischen Roggen-Fruchtschalen erhaltene Masse III liefert nach NaOH- und HCl-Extraktion 200 l Lösung VIII, deren Trockenrückstand nach Standardisierung auf einen Wert von 1,7 % eingestellt worden ist. Dieser Extrakt ist ohne weiteres mit einem der in Beispiel 1 beschriebenen Roggen-Plazenta-Extrakte auf Zellkulturbasis kombinierbar, kann aber auch ohne Roggen-Plazenta-Extrakt-Zusatz eingesetzt werden.

Die Kennzahlen eines 1 : 1 enthaltenden Roggen-Plazenta-Extrakts sind beispielsweise folgende:

pH 6,70; Trockenrückstand 1,71 %; N: 1,89 mg/ml; Warburg-Faktor 1,95; weitere Daten entsprechend Tabelle 1.

Beispiel 7

Herstellung eines Roggen-Plazente, Extrakts aus einer Zellkultur, gewonnen aus Plazenta-haltigen Roggen-Fruchtschalen gemäß Beispiel 1 und gemäß Beschreibungen im Text

Einzelheiten sind aus der Übersicht zu 1.1 des Beispiels 1 zu entnehmen.

Der Beweis dafür, dass die Zellen der Ausgangskultur der Roggen-Plazenten identisch sind mit denjenigen der gezüchteten Zellmasse, wurde mit Hilfe eines Plasmids und DNA-Analyse geführt.

Beispiel 8

Herstellung eines Weizen-Plazenta-Extrakts (Weizen: Triticum vulgare) via Zellkultur

Ausgangskultur: Eine analog zu dem obigen Beispiel 4 im Jahre 1991 in der UFSM angesetzte Weizen-Plazenta-Kultur, aus der in der vorstehend beschriebenen Weise eine ausreichende Menge an Zellmasse gezüchtet wurde.

Anschließend wurde die Zellmasse mit einem Gemisch von Propylenglycol/Wasser/Ethanol behandelt und das Lösungsmittel wurde unter einem Vakuum von 0,1 Torr teilweise abgezogen. Der verbliebene wässrige Destillationsrückstand wurde zur Deproteinisierung mit Säuren des Citronensäurecyclus gefällt, auf pH 6,7 eingestellt und dialysiert. Der Trockenrückstand wurde auf 2,0 bis 2,3 % eingestellt, nachdem die gewünschte Menge an Konservierungsmittel hinzugefügt worden war. Der End-pH-Wert betrug 6,7. Eine Sterilfiltration wurde in bekannter Weise durchgeführt.

Daten hinsichtlich der Wirksamkeit des Weizen-Plazenta-Extrakts:

| | |
|---|---|
| Atmungssteigerung von Leberhomogenat | |
| Warburg-Faktor | 2,3 |
| Beschleunigung der Metamorphose bei Xenopus leavis | 5 Tage |
| Padberg-Methode: | |
| Rauigkeitswerte: (Testcreme): (Kontrolle-Creme) | 59:90 |
| Patch-Test: Testcreme wie Kontroll-Creme | 0 |

**Patentansprüche**

1. Verfahren zur Herstellung von Plazenta-Extrakten von Cerealien aus der Gruppe Weizen, Reis, Gerste, Roggen, Hafer, Sorghum (Hirse), Triticale und Dinkel aus Plazenta-Zellen von speziell hergestellten oder handelsüblichen Plazenta-Zelllinien der genannten Cerealien,
**gekennzeichnet durch** die folgenden Stufen:

a) Aktivierung der gewählten Plazenta-Zelllinie einer der Cerealien aus der Gruppe Weizen, Reis, Gerste, Roggen, Hafer, Sorghum (Hirse), Triticale und Dinkel, in einem Wasserbad von vorzugsweise 25 bis 30 °C,
b) Zugabe eines vorgewärmten, für die jeweils gewählte Zelllinie vorgeschriebenen Kulturmediums unter Vermischung und anschließendes Inkubieren in an sich bekannter Weise, vorzugsweise bei etwa 37°C, und vorzugsweise in einer etwa 5 % $CO_2$ enthaltenden Atmosphäre,
c) Gewinnung der dabei gebildeten Zellen als Ausgangsmaterial für Großkulturen in Suspension **durch** Dekantieren des Nährmediums, Waschen der entstandenen Zellmasse mit einem geeigneten Puffer und Aufnahme der Zellen in einem kompletten Medium unter Einstellung einer für die Massenproduktion geeigneten Verdünnung,
d) Kultivierung der Zellen in Suspensionskultur im Großmaßstab auf an sich bekannte Weise **durch** Beimpfen einer Kultivierungsapparatur mit einer Zellsuspension in einem sterilen $CO_2$/Luft-Gemisch,
wobei **durch** Einbringen eines Plasmids und **durch** DNA-Analyse gezeigt werden kann, dass die in Masse gezüchteten Zellen denjenigen der Anfangs-Zellkultur entsprechen,
e) Herstellung eines Extrakts **durch** Behandlung des suspendierten geernteten Zelimaterials in einem organischen Lösungsmittel und/oder Wasser, Gewinnung der wäßrigen Phase vorzugsweise **durch** Zentrifugieren, Entfernung von restlichen Lipidanteilen (Rückstand I) aus der wäßrigen Phase, gegebenenfalls **durch** mehrmaliges Behandeln mit dem organischen Lösungsmittel unter Bildung eines wäßrigen Rohextrakts, gegebenenfalls in Form einer Suspension,
f) Reinigung bzw. Behandlung des Rohextrakts je nach Ausgangsmaterial; und
g) Sterilfiltration des Extrakts, vorzugsweise unter Verwendung eines 0,2 $\mu$m (Millipore®-Filters oder einer PALL-Kerze nach Einstellung des Extrakts auf den gewünschten pH-Wert, vorzugsweise 5,0 bis 7,2.

2. Verfahren nach Anspruch 1, **dadurch gekennzeichnet, dass** eine in der Stufe (d) gewonnene pflanzliche Zellmasse in Ethylether oder Chloroform suspendiert und etwa 8 bis etwa 15 Tage lang bei etwa -10 bis etwa -20°C im Kälteraum stehen gelassen und dann zur Gewinnung der wäßrigen Phase zentrifugiert wird.

3. Verfahren nach Anspruch 1 oder 2, **dadurch gekennzeichnet, dass** die letzten Spuren von Ether oder Chloroform aus dem Dialysat (Außenlösung) in der Stufe (f) durch Stickstoff-Durchblasen bei pH 7,2 bis 7,5 entfernt werden.

4. Verfahren nach mindestens einem der Ansprüche 1 bis 3, **dadurch gekennzeichnet, dass** als Zelllinie eine Plazenta-Zelllinie aus der Fruchtschale (Pericarp), der Samenschale (Testa) oder der proteinreichen Aleuronschicht von Cerealien aus der Gruppe Hafer, Gerste, Reis, Triticale, Roggen, Weizen, Dinkel und Sorghum (Hirse) verwendet wird.

5. Verfahren nach mindestens einem der Ansprüche 1 bis 4, **dadurch gekennzeichnet, dass** dann, wenn es auf das Zellwandmaterial ankommt, das in Wasser oder in einem organischen Lösungsmittel suspendierte Zellmaterial in der Stufe (e) in einem Ultraschallgenerator zerbrochen und das aufgeschlossene Material nach dem Zentrifugieren und Waschen mit Wasser durch chemische und/oder enzymatische Partialhydrolyse aufgeschlossen und dialysiert wird.

6. Verfahren nach mindestens einem der Ansprüche 1 bis 5, **dadurch gekennzeichnet, dass** zur Herstellung von Pflanzen-Plazenta-Extrakten aus Zelllinlen der Cereallen solche Ausgangsmateriallen verwendet werden, die keiner Genmanipulation unterzogen worden sind und die frei sind von unerwünschten Substanzen, speziell immunogenen und/oder pathogenen Proteinen (Prionen) und Proteinabbauprodukten, und in denen sich keine Insektizid-Rückstände nachweisen lassen.

7. Verfahren nach mindestens einem der Ansprüche 1 bis 6, **dadurch gekennzeichnet, dass** ein oder mehrere von aus einer oder mehreren unterschiedlichen Cereallen-Plazenta-Zelllinlen gewonnenen, sterilfiltrierten Plazenta-Extrakten hergestellt und gegebenenfalls kombiniert wird (werden) mit einer oder mehreren aus einem oder mehreren unterschiedlichen tierischen Zelllinien gewonnenen, sterilfiltrierten tierischen Organextrakten sowie gegebenenfalls mit einem oder mehreren aus einem oder mehreren verschiedenen, synthetisch hergestellten tierischen Organextrakten und/oder mit einem oder mehreren verschiedenen, aus frischen tierischen Organen hergestellten tierischen Organextrakten und/oder gegebenenfalls mit einem oder mehreren aus einem oder mehreren verschiedenen, aus frischen Pflanzenorganen bzw. -tellen hergestellten Pflanzenextrakten.

8. Verfahren nach Anspruch 7, **dadurch gekennzeichnet, dass** das Gewichtsverhältnis zwischen dem (den) pflanzlichen Organ-Extrakt(en) und dem (den) tierischen Organ-Extrakt(en) (1 bis 99) ; (99 bis 1), vorzugsweise (1 bis 50) : (50 bis 1), insbesondere (1 bis 20) : (99 bis 80), beträgt.

9. Verwendung der Cerealien-Plazenta-Extrakte und Extraktgemische, hergestellt nach mindestens einem der Ansprüche 1 bis 8, zur Herstellung eines Präparats mit Radikalfängerwirkung, insbesondere für kosmetische und/oder technische Zwecke.

10. Verwendung der Cerealien-Plazenta-Extrakte und Extraktgemische, hergestellt nach mindestens einem der Ansprüche 1 bis 8, zur Herstellung einer kosmetischen Formulierung.

11. Verwendung nach Anspruch 10 zur Herstellung einer kosmetischen Formulierung mit die Melaninbildung hemmenden Eigenschaften (Whitening Effect).

12. Verwendung der Cerealien-Plazenta-Extrakte und Extraktgemische, hergestellt nach mindestens einem der Ansprüche 1 bis 8, zur Herstellung eines medizinischen Präparats für die topische, subkutane, intravenöse, Intramuskuläre oder orale Applikation zum Zwecke der äußeren oder inneren Wundheilung, zur Behandlung von Arteriosklerose oder von Strahlungsschäden.

13. Verwendung der Cereallen-Plazenta-Extrakte und Extraktgemische, hergestellt nach mindestens einem der Ansprüche 1 bis 8, zur Herstellung eines medizinischen Präparats für die Stärkung des Immunsystems, für die Aktivierung des Zellstoffwechsels oder die Behandlung gastroenterologischer Erkrankungen, insbesondere Ulcera.

14. Verwendung der Cerealien-Plazenta-Extrakte und Extraktgemische, hergestellt nach mindestens einem der Ansprüche 1 bis 8, zur Herstellung eines Präparats mit aphrodisiakatischer Wirkung.

15. Verwendung der Cerealien-Plazenta-Extrakte und Extraktgemische, hergestellt nach mindestens einem der Ansprüche 1 bis 8, und der darin enthaltenen Rohextrakte und Vorstufen zur Stabilisierung und Haltbarmachung von gefärbten Geweben und Farbanstrichen, insbesondere Lackierungen.

16. Verwendung der Cerealien-Plazenta-Extrakte und Extraktgemische, hergestellt nach mindestens einem der Ansprüche 1 bis 8, zur Herstellung von Kulturlösungen, Nährlösungen und diätetischen Lösungen.

17. Verwendung nach Anspruch 16 zur Herstellung von Nährlösungen und diätetischen Lösungen in sie (verdünnt oder unverdünnt) enthaltenden Kapseln für die direkte orale Einnahme, insbesondere als Nahrungsergänzungsmittel.


**Claims**

1. A process for preparing placenta extracts of cereals, selected from the group consisting of wheat, rice, barley, rye, oats, sorghum (millet), triticale and spelt, from placenta cells of specifically prepared or commercially available placenta cell lines of the said cereals, **characterized by** the following steps:

   a) activation of the selected placenta cell line of one of the cereals, selected from the group consisting of wheat, rice, barley, rye, oats, sorghum (millet), triticale and spelt, in a water bath at a temperature of preferably from 25 to 30 °C,
   b) addition of a pre-heated culture medium which is prescribed for the selected cell line by mixing it therewith and then incubating it in a manner known per se, preferably at a temperature of about 37 °C and preferably in an atmosphere containing about 5 % $CO_2$,
   c) separating and using the obtained cells as starting material for mass cultures in suspension by decanting the culture medium, washing the produced cell mass with a suitable buffer and introducing the cells into a complete medium and adjusting a dilution which is suitable for the mass production,
   d) cultivating the cells in suspension culture in a large scale in a manner known per se by inoculating a cell suspension in a sterile $CO_2$/air mixture into a cultivating apparatus,
   wherein by introducing a plasmide and by DNA analysis it can be shown that the cells cultivated in large scale correspond to those of the initial cell culture;
   e) preparation of an extract by treating the suspended collected cell material in an organic solvent and/or in water, separating the aqueous phase, preferably by centrifugating, removing residual lipid moieties (residue I) from the aqueous phase, optionally by multiple treatment with the organic solvent, for forming an aqueous raw extract, optionally in the form of a suspension,
   f) purification or treatment of the raw extract depending on the starting material; and
   g) sterile filtration of the extract, preferably by using a 0.2 $\mu$m Millipore® filter or a PALL-candle, after having adjusted the pH of the extract to a desired value of preferably 5.0 to 7,2.

2. The process according to claim 1, **characterized in that** a vegetal cell mass obtained in step (d) is suspended in ethyl ether or chloroform and stored for about 8 to about 15 days at a temperature of about -10 °C to about -20°C in a cooling room and then centrifugated for separating the aqueous phase.

3. The process according to claim 1 or 2, **characterized in that** the last traces of ether or chloroform are removed from the dialysate (outer solution) in step (f) by introducing nitrogen at pH 7.2 to 7.5.

4. The process according to at least one of claims 1 to 3, **characterized in that** as a cell line a placenta cell line of the pericarp (Fruchtschale), the testa (Samenschale) or the protein-rich aleuron layer (Aleuronschicht) of cereals, selected from the group consisting of wheat, rice, barley, rye, oats, sorghum (millet), triticale and spelt, is used.

5. The process according to at least one of claims 1 to 4, **characterized in that**, if the cell wall material is important, the cell material suspended in water or in an organic solvent in step (e) is broken open in an ultrasonic generator and the obtained material after having been centrifugated and washed with water is treated by chemical and/or enzymatic partial hydrolysis and dialyzed.

6. The process according to at least one of claims 1 to 5, **characterized in that** for preparing vegetal placenta extracts from cell lines of cereals such starting materials are used which have not been subjected to a gene manipulation and which are free of undesired substances, especially free of immunogenic and/or pathogenic proteins (prions) and protein degradation products, and wherein no residues of insecticides can be detected.

7. The process according to at least one of claims 1 to 6, **characterized in that** one or more sterile filtrated vegetal extracts, which have been produced from one or more different vegetal cell lines, are prepared and optionally combined with one or more sterile filtrated animal organ extracts, which have been produced from one or more different animal cell lines, and optionally combined with one or more different, synthetically prepared animal organ extracts, and/or with one or more different animal organ extracts, which have been produced from fresh animal organs, and/or optionally with one or more vegetal extracts which have been produced from one or more different fresh organs or parts of plants.

8. The process according to claim 7, **characterized in that** the weight ratio between the vegetal placenta extract(s) and the animal organ extract(s) is (1 to 99) : (99 to 1), preferably (1 to 50) : (50 to 1), especially (1 to 20) : (99 to 80).

9. Use of the cereals placenta extracts and extract mixtures according to at least one of claims 1 to 8 for preparing a composition having a radical removing effect, in particular for cosmetic and/or technical purposes.

10. The use of the cereals placenta extracts and extract mixtures prepared according to at least one of claims 1 to 8 for preparing a cosmetic formulation.

11. The use according to claim 10 for preparing a cosmetic formulation having properties which inhibit the melamine formation (whitening effect).

12. The use of the cereals placenta extracts and extract mixtures prepared according to at least one of claims 1 to 8 for preparing a medical preparation for the topical, subcutaneous, intravenous or intramuscular administration for the healing of outer or inner wounds, for treatment of arteriosclerosis or of radiation damages.

13. The use of the cereals placenta extracts and extract mixtures prepared according to at least one of claims 1 to 8 for preparing a medical preparation for improving the immune system, for activating of the cell metabolism or for the treatment of gastroenterological diseases, in particular ulcera.

14. The use of the the cereals placenta extracts and extract mixtures prepared according to at least one of claims 1 to 8 for preparing a composition having an aphrodisiacatic effect.

15. The use of the the cereals placenta extracts and extract mixtures prepared according to at least one of claims 1 to 8 and of the raw extracts and precursors contained therein for stabilizing and improving durability of colored fabrics and color paints, especially lacquer works.

16. The use of the cereals placenta extracts and extract mixtures prepared according to at least one of claims 1 to 8 for preparing cultivation solutions, nutrition solutions and dietetic solutions.

17. The use according to claim 16 for preparing nutrition solutions and dietetic solutions in the form of capsules containing them in an undiluted or diluted form for oral administration, in particular as foodstuff complements.

**Revendications**

1. Procédé de fabrication d'extraits de placenta de céréales issues du groupe blé, riz, orge, seigle, avoine, sorgho (millet), triticale et épeautre, à partir de cellules de placenta de lignées cellulaire de placenta, courantes ou fabriquées à cet effet, des céréales citées,
   **caractérisé par** les étapes suivantes:

   a) activation de la lignée cellulaire de placenta sélectionnée d'une des céréales du groupe blé, riz, orge, seigle, avoine, sorgho (millet), triticale et épeautre dans un bain-marie de préférence de 25 à 30°C,
   b) ajout d'un milieu de culture préchauffé et prescrit pour la lignée cellulaire sélectionnée concernée par mélange, puis incubation d'une manière connue en soi, de préférence à environ 37°C et de préférence dans une atmosphère contenant environ 5 % de $CO_2$,

c) extraction des cellules ainsi formées afin de les employer comme matériau de départ pour de grandes cultures en suspension par décantation du milieu nutritif, nettoyage de la masse cellulaire obtenue au moyen d'un tampon adapté et dépôt des cellules dans un milieu complet en réglant une dilution adaptée à la production en masse,

d) mise en culture à grande échelle des cellules dans une culture en suspension, d'une manière connue en soi, par inoculation d'un appareil de culture à partir d'une suspension de cellules dans un mélange stérile $CO_2$/air, sachant que l'introduction d'un plasmide et l'analyse de l'ADN permettent de démontrer que les cellules cultivées en masse correspondent à celles de la culture de cellules de départ,

e) fabrication d'un extrait par traitement du matériel cellulaire en suspension récolté, dans un solvant organique et/ou de l'eau, extraction de la phase aqueuse, de préférence par centrifugation, suppression des lipides résiduels (résidu I) de la phase aqueuse, le cas échéant par traitement répété à partir d'un solvant organique en formant un extrait brut aqueux , le cas échéant sous la forme d'une suspension,

f) rinçage et/ou traitement de l'extrait brut en fonction du matériau de départ ; et

g) filtration stérilisante de l'extrait, de préférence en utilisant un filtre (Millipore®) de 0,2 $\mu$m ou une bougie PALL, après avoir réglé l'extrait à la valeur de pH souhaitée, de préférence comprise entre 5,0 et 7,2.

**2.** Procédé suivant la revendication 1, **caractérisé en ce qu'**une masse cellulaire végétale obtenue au cours de l'étape (d) est en suspension dans de l'éther éthylique ou du chloroforme et laissée environ 8 à environ 15 jours dans une chambre froide dont la température est comprise entre environ -10 et environ -20°C puis centrifugée afin d'extraire la phase aqueuse.

**3.** Procédé suivant la revendication 1 ou 2, **caractérisé en ce que** les dernières traces d'éther ou de chloroforme sont supprimées du dialysat (solution extérieure), au cours de l'étape (f), en faisant barboter de l'azote à une valeur de pH comprise entre 7,2 et 7,5.

**4.** Procédé suivant au moins l'une des revendications 1 à 3, **caractérisé en ce qu'**on emploie comme lignée cellulaire une lignée cellulaire de placenta issue du tégument du fruit (péricarpe), du tégument du grain (testa) ou de la couche d'aleurone protéique de céréales provenant du groupe avoine, orge, riz, triticale, seigle, blé, épeautre et sorgho (millet).

**5.** Procédé suivant au moins l'une des revendications 1 à 4, **caractérisé en ce que**, lorsque c'est le matériau de la paroi cellulaire qui importe, le matériau cellulaire suspendu dans de l'eau ou dans un solvant organique est fragmenté au cours de l'étape (e) dans un générateur à ultrasons et que le matériau désagrégé est désagrégé par hydrolyse partielle chimique et/ou enzymatique et dialysé après la centrifugation et le rinçage à l'eau.

**6.** Procédé suivant au moins l'une des revendications 1 à 5, **caractérisé en ce qu'**on utilise, pour la fabrication d'extraits de placenta végétaux à partir de lignées cellulaires des céréales, des matériaux de départ qui n'ont fait l'objet d'aucune manipulation génétique, qui ne contiennent aucune substance indésirable, en particulier aucune protéine immunogène et/ou pathogène (prions) ni aucun produit de la protéolyse, et dans lesquels ne se trouve aucun résidu d'insecticide.

**7.** Procédé suivant au moins l'une des revendications 1 à 6, **caractérisé en ce qu'**un ou plusieurs extrait(s) de placenta stérilisé(s) par filtrage et obtenu(s) à partir d'une ou de plusieurs différente(s) lignée(s) cellulaire(s) de placenta de céréales est(sont) fabriqué(s) et, le cas échéant, combiné(s) avec un ou plusieurs extrait(s) organique(s) animal (aux) stérilisé(s) par filtrage et obtenu(s) à partir d'une ou de plusieurs lignée(s) cellulaire(s) animale(s) différente (s) ainsi que, le cas échéant, avec un ou plusieurs extrait(s) organique(s) animal(aux) fabriqué(s) par synthèse à partir d'un ou de plusieurs organe(s) différent(s) et/ou avec un ou plusieurs extrait(s) organique(s) animal(aux) différent(s), fabriqué(s) à partir d'organes animaux frais, et/ou, le cas échéant, avec un ou plusieurs extrait(s) végétal (aux) fabriqué(s) à partir d'un ou de plusieurs organe(s) ou élément(s) végétal(aux) frais différent(s).

**8.** Procédé suivant la revendication 7, **caractérisé en ce que** le rapport du poids entre l(es)'extrait(s) organique(s) végétal(aux) et l(es)'extrait(s) organique(s) animal(aux) s'élève à (1 à 99) : (99 à 1), de préférence (1 à 50) : (50 à 1), en particulier (1 à 20) : (99 à 80).

**9.** Utilisation des extraits de placenta de céréales et des mélanges d'extraits, fabriqués suivant au moins l'une des revendications 1 à 8, afin de produire une préparation antiradicalaire, en particulier à des fins cosmétiques et/ou techniques.

**10.** Utilisation des extraits de placenta de céréales et des mélanges d'extraits, fabriqués suivant au moins l'une des revendications 1 à 8, afin de produire une formule cosmétique.

**11.** Utilisation suivant la revendication 10 afin de produire une formule cosmétique ayant des propriétés qui empêchent la formation de mélanine (effet blanchissant).

**12.** Utilisation des extraits de placenta de céréales et des mélanges d'extraits, fabriqués suivant au moins l'une des revendications 1 à 8, afin de produire une préparation médicale destinée à une administration topique, sous-cutanée, intraveineuse, intramusculaire ou orale à des fins de cicatrisation externe ou interne, de traitement de l'artériosclérose ou de dommages résultant de rayonnements.

**13.** Utilisation des extraits de placenta de céréales et des mélanges d'extraits, fabriqués suivant au moins l'une des revendications 1 à 8, afin de produire une préparation médicale permettant de renforcer le système immunitaire, d'activer le métabolisme cellulaire ou de traiter des maladies gastro-entérologiques, en particulier les ulcères.

**14.** Utilisation des extraits de placenta de céréales et des mélanges d'extraits, fabriqués suivant au moins l'une des revendications 1 à 8, afin de produire une préparation ayant des effets aphrodisiaques.

**15.** Utilisation des extraits de placenta de céréales et des mélanges d'extraits, fabriqués suivant au moins l'une des revendications 1 à 8, et des extraits bruts et précurseurs qu'ils contiennent afin de stabiliser et de conserver des tissus colorés ainsi que des peintures, en particulier des vernis.

**16.** Utilisation des extraits de placenta de céréales et des mélanges d'extraits, fabriqués suivant au moins l'une des revendications 1 à 8, afin de produire des solutions de culture, des solutions nutritives et des solutions diététiques.

**17.** Utilisation suivant la revendication 16 afin de produire des solutions nutritives et des solutions diététiques en gélules les contenant (diluées ou non diluées) et destinées à une administration orale directe, en particulier comme compléments nutritifs.

Fig. 1

Gas

Saugflasche

Gummiverschlußkappe

Schlauch zur Erneuerung
des Mediums

Kulturmedium

Probenschlauch

Rührstab

Magnetrührer

Universalbehälter

Fig. 2

**Fig. 3**

Rohrschlangen-Kultivator IGV

**Fig. 4a**      Corneometer CM 820 PC

**Fig. 4b**      Meßfühler vom Corneometer CM 820 PC

Fig. 5: Hautfeuchtigkeitsmessungen